# EUROPEAN PATENT APPLICATION

(11) **EP 2 108 701 A1**
(43) Date of publication of application: **14.10.2009**
(21) Application number: 08007109.5
(22) Date of filing: 10.04.2008
(51) Int. Cl.: C12N 15/11, A61K 39/00, G01N 33/68, C07K 14/705

(54) **Methods involving MS4A12 and agents targeting MS4A12 for therapy, diagnosis and testing**

(71) Applicant: Ganymed Pharmaceuticals AG, 55131 Mainz (DE); Johannes Gutenberg-Universität Mainz, 55122 Mainz (DE)
(72) Inventor: Sahin, Ugur, 55116 Mainz (DE); Türeci, Özlem, 55116 Mainz (DE); Koslowski, Michael, 55116 Mainz (DE)
(74) Representative: Schnappauf, Georg

(57) **Abstract**

The invention relates to methods involving MS4A12 and agents targeting MS4A12 which are useful for therapy, diagnosis and testing of disease states, in particular cancer diseases. In particular, the invention relates to agents which reduce or inhibit expression or activity of MS4A12, compositions containing these agents, and methods for reducing, determining, or increasing the responsiveness of a cell towards EGF.

## Description

The invention relates to methods and agents which are useful for therapy, diagnosis and testing of disease states, in particular cancer diseases. In particular, the invention relates to the inhibition and determination of MS4A12 activity, for example in neoplastic cells of a cancer patient, in particular in cells of colon cancer.

Despite interdisciplinary approaches and exhaustive use of classical therapeutic procedures, cancers are still among the leading causes of death.

Antibody based cancer therapies have been successfully introduced into the clinic and have emerged over the last decade as the most promising therapeutics in oncology (Glennie et al., Drug Discov. Today 8: 503-10, 2003). Hematological malignancies provided a successful testing ground for therapeutic concepts based on monoclonal antibodies (mAbs). In these diseases antigens, which are expressed exclusively on a particular lineage of hematopoetic cells, such as CD20, CD22, CD25, CD33 and CD52 (Countouriotis et al., Stem Cells 20: 215-29, 2002; Dillman et al., Cancer Pract. 9: 71-80, 2001; Leget et al., Curr. Opin. Oncol. 10: 548-51, 1998), have proven as useful therapeutic targets. Most prominent amongst these are anti-CD20 mAbs as they have revolutionized the treatment of lymphoma patients. The chimeric anti-CD20 mAb rituximab has achieved response rates of approximately 40% to 60% in patients with multiply relapsed indolent B-cell lymphomas and has become the first mAb approved by the United States Food and Drug Administration for the treatment of cancer (Leget et al., Curr. Opin. Oncol. 10: 548-51, 1998). The spectrum of lymphoproliferative disorders eligible for CD20-targeting therapy is still growing (Leonard et al., Hematology, Am. Soc. Hematol. Educ. Program 335-9, 2005).

Two key characteristics contribute to the clinical success of CD20 as an antibody target. First, this target provides an optimal therapeutic window, as it is a highly selective differentiation marker for the B-cell lineage. CD20 expression is neither detectable on any other normal cell type nor on the stem cell precursor of the B-cell lineage (Cartron et al., Blood 104: 2635-42, 2004). Accordingly, there is no severe dose-limiting toxicity and normal B-lymphocyte levels are eventually restored by target-negative stem cells. B-cell malignancies, which in general preserve expression of this differentiation antigen, can be treated successfully. The second peculiarity is that in comparison to other mAbs against hematopoetic differentiation antigens, anti-CD20 antibodies are of unusual high cytocidal potency (Cragg et al., Blood 101: 1045-52, 2003). At least in vitro, they mediate both complement-dependent cytotoxicity (CDC) and antibody-dependent cell-mediated cytotoxicity (ADCC). Efficient recruitment of cellular and soluble immune effectors seems to be closely linked to the characteristic membrane topology of the CD20 molecule accounting for a high lateral mobility of antibody/antigen complexes and a short distance between the target epitope and the lipid bilayer (Cragg et al., Blood 101: 1045-52, 2003; Cragg et al., Blood 103: 2738-43, 2004; Johnson et al., Semin. Oncol. 30: 3-8, 2003). In addition to recruiting immune effectors anti-CD20 mAbs are able to mediate growth arrest and apoptosis in certain cell lines (Deans et al., Immunology 107: 176-82, 2002; Shan et al., Blood 91: 1644-52, 1998; Shan et al., Cancer Immunol. Immunother. 48: 673-83, 2000; Unruh et al., Immunology 116: 223-32, 2005). Mechanisms by which anti-CD20 mAbs interfere with proliferation and survival remained unknown until recently, when CD20 was established as a component of a SOC entry pathway activated by the B-cell receptor.

Intracellular Ca²⁺ is an essential regulator of cell function. Signaling through growth receptors leads to rapid release of Ca²⁺ from intracellular stores into the cytoplasm. Store depletion activates Ca²⁺ influx across the plasma membrane, replenishing empty stores and providing a sustained increase in the concentration of cytoplasmic free Ca²⁺ ([Ca²⁺]_{c}). This Ca²⁺ influx has been termed capacitative or store-operated Ca²⁺ (SOC) entry and is essential for regulating diverse cellular responses to receptor-mediated stimuli, including survival and proliferation (Berridge, J. Physiol. 499: 291-306, 1997; Berridge et al., Nature 395: 645-8, 1998; Berridge et al., Nat. Rev. Mol. Cell Biol. 1: 11-21, 2000). In line with the role of CD20 in modulation of Ca²⁺ signaling, it has been shown that Ca²⁺ influx is essential for Rituximab induced antiproliferative and apoptotic effects (Shan et al., Cancer Immunol. Immunother. 48: 673-83, 2000; Ghetie et al., Blood 97: 1392-98, 2001; Hofmeister et al., Blood Cells Mol. Dis. 26: 133-43, 2000).

Lessons learned from anti-CD20 antibodies and rationales developed in hematological diseases may empower the design of rational mAb therapies in solid cancer with still high medical need. However, for solid tumors targets of analogous potency are not yet available.

It was the object of the present invention to identify such target structures and utilizing them in the therapy, diagnosis and testing of cancers.

The object of the present invention is achieved by the subject matter of the claims.

A data mining strategy for discovery of differentiation genes of colonic epithelia exploitable as targets for therapeutic antibodies in colon cancer led to MS4A12, a close relative of CD20. The inventors have shown that MS4A12 is a colonocyte specific differentiation gene involved in SOC entry, which functions as a novel modulator of EGFR signaling. The data provided by the inventors give unexpected insights into the physiology of colonic epithelial cells and into the biology of certain cancer diseases, in particular colon cancer, and provide a novel target for the treatment of such cancer diseases.

In one aspect, the invention provides agents which reduce or inhibit expression or activity of MS4A12 in a cell.

Preferably, the agent is specific for MS4A12. The term "agent specific for MS4A12" refers to an agent that reduces or inhibits expression or activity of MS4A12 to a higher extent than it reduces or inhibits expression or activity of another gene product. Preferably, expression or activity of MS4A12 is reduced or inhibited to an at least 1.2 fold, at least 1.5 fold, at least 2 fold, at least 3 fold, at least 5 fold, at least 10 fold, at least 50 fold, at least 100 fold, or at least 1000 fold greater extent by the agent which reduces or inhibits expression or activity of MS4A12 of the invention than expression or activity of any other gene product.

An "agent which reduces or inhibits expression of MS4A12 in a cell" may reduce or inhibit transcription of the MS4A12 gene, reduce or inhibit procession of the MS4A12 pre-mRNA (such as splicing, poly-adenylation, capping, etc.), reduce the half-life of the MS4A12 mRNA, and/or reduce or inhibit translation of the MS4A12 mRNA into MS4A12 protein. In particular, the amount of MS4A12 protein synthesized from an MS4A12 gene in a cell is reduced in the presence of the agent which reduces or inhibits expression of MS4A12 in a cell.

An "agent which reduces or inhibits activity of MS4A12 in a cell" may reduce or inhibit one or more activities of MS4A12. The term "activity of MS4A12" relates to any activity of MS4A12 in a cell.

Preferably, the activity of MS4A12 is relevant for cancer diseases such as colon cancer and colon cancer metastasis. In particular, it is involved in formation, progression and/or maintenance of tumors or tumor metastases such as colon tumors or colon tumor metastases. Preferably, the activity of MS4A12 is selected from the group consisting of increasing or enabling cell growth, proliferation, viability, cell cycle progression, migration, chemotaxis and invasion.

In specific embodiments, the activity of MS4A12 involves increasing or enabling store-operated Ca²⁺ entry in a cell.

It is to be understood that reduction or inhibition of expression of MS4A12 in a cell preferably will also result in reduction or inhibition of activity of MS4A12 in said cell as discussed above.

In a particular embodiment, the activity of MS4A12 is increasing or enabling responsiveness of a cell towards EGF. According to the invention, "responsiveness of a cell towards EGF" refers to the ability of a cell to exhibit one or more responses upon the contact with EGF. The response may be any response such as a biochemical response such as the change in concentration of a molecule, e.g. a calcium ion, in particular depletion of intracellular Ca²⁺ stores or store-operated Ca²⁺ entry, or the modification of a molecule, e.g. phosphorylation, de-phosphorylation, glycosylation or de-glycosylation of a protein or methylation or de-methylation of a nucleic acid. Furthermore, the response may be a signaling activity such as the activation or inhibition of a signaling pathway, in particular the activation of a signaling pathway resulting in depletion of intracellular Ca²⁺ stores or resulting in store-operated Ca²⁺ entry, or the secretion of a signaling molecule, e.g., a hormone. A further example of the response is a response at the genetic level such as activation or inhibition of the expression of a gene, for example the activation of an oncogene or the inactivation of a tumor suppressor gene. Furthermore, the response may be a change in one or more cellular characteristics such as a change in cell morphology or an increase of cell growth, proliferation, cell cycle progression, migration, chemotaxis or invasion.

Preferred EGF-induced responses are EGF-induced growth, EGF-induced proliferation, EGF-induced cell cycle progression, EGF-induced migration, EGF-induced chemotaxis and EGF-induced invasion of the cell and a combination thereof.

Likewise, "a cell responsive towards EGF" is a cell exhibiting any of the responses described above when brought into contact with EGF.

In a preferred embodiment, the cell expresses MS4A12. In an even more preferred embodiment, the cell also expresses an EGF receptor.

In one embodiment, the agent which reduces or inhibits expression or activity of MS4A12 of the invention reduces or inhibits expression or activity of human MS4A12, preferably human MS4A12 having the amino acid sequence of SEQ ID NO: 2.

In one embodiment, the agent comprises, and preferably consists of an siRNA which specifically targets and causes RNAi-induced degradation of mRNA from MS4A12 genes, so that the protein product of the MS4A12 gene is not produced or is produced in reduced amounts.

Preferably, the siRNA according to the invention comprises a sense RNA strand and an antisense RNA strand, wherein the sense and antisense RNA strands form an RNA duplex. Preferably, the sense RNA strand comprises a nucleotide sequence substantially identical to a target sequence of about 19 to about 25 contiguous nucleotides in a nucleic acid coding for MS4A12, preferably an mRNA coding for MS4A12. Preferably, the siRNA of the invention is isolated.

In one embodiment, the siRNA of the invention is assembled from two nucleic acid fragments wherein one fragment comprises the sense strand and the second fragment comprises the antisense strand of said siRNA molecule. In a further embodiment of the siRNA of the invention, the sense and antisense RNA strands forming the RNA duplex are covalently linked, for example by a single-stranded hairpin.

The siRNA of the invention preferably further comprises non-nucleotide material. In a further embodiment of the siRNA of the invention, the sense and antisense RNA strands are stabilized against nuclease degradation.

Preferably, the siRNA of the invention further comprises a 3'-overhang preferably comprising from 1 to about 6 nucleotides, more preferably about 2 nucleotides.

In one embodiment of the siRNA of the invention, the sense RNA strand comprises a first 3'-overhang, and the antisense RNA strand comprises a second 3'-overhang, wherein preferably the first and second 3'-overhangs independently comprise from 1 to about 6 nucleotides. More preferably, the first 3'-overhang comprises a dinucleotide and the second 3'-overhang comprises a dinucleotide, preferably dideoxythymidylic acid or diuridylic acid.

In one embodiment of the siRNA of the invention, the 3'-overhang is stabilized against nuclease degradation.

In particular embodiments of the siRNA of the invention, the target sequence has the nucleotide sequence of nucleotide positions 344-363 of SEQ ID NO: 1, or the nucleotide sequence of nucleotide positions 247-266 of SEQ ID NO: 1. Thus, the target sequence may have the nucleotide sequence of SEQ ID NO: 3 or SEQ ID NO: 6. In further particular embodiments of the siRNA of the invention, the sense RNA strand has the sequence of SEQ ID NO: 4 and the antisense RNA strand has the sequence of SEQ ID NO: 5, or the sense RNA strand has the sequence of SEQ ID NO: 7 and the antisense RNA strand has the sequence of SEQ ID NO: 8.

In a further embodiment, the agent comprises, and preferably consists of an antisense nucleic acid which hybridizes selectively with a nucleic acid coding for MS4A12. In particular, the antisense nucleic acid hybridizes specifically with an MS4A12 gene and/or an MS4A12 mRNA and thereby prevents or reduces transcription of the MS4A12 gene and/or translation of the MS4A12 mRNA.

Preferably, the antisense nucleic acid is substantially complementary to a target sequence of about 20 to about 30 contiguous nucleotides in a nucleic acid coding for MS4A12, preferably a gene or mRNA coding for MS4A12. Preferably, the antisense nucleic acid of the invention is isolated.

The target sequence can be located in any part of the MS4A12 gene or MS4A12 mRNA as long as the desired effect is achieved. For example, the antisense nucleic acid of the invention may hybridize to the promoter or the transcription initiation site of the MS4A12 gene, a splice donor or acceptor site of the MS4A12 pre-mRNA, the ribosome binding site or the translation initiation site of the MS4A12 mRNA, or the MS4A12 coding region.

The antisense nucleic acid of the invention preferably further comprises non-nucleotide material. In a further embodiment, the antisense nucleic acid of the invention is stabilized against nuclease degradation.

In a further embodiment, the agent comprises, and preferably consists of an antibody which binds selectively to MS4A12. By binding to MS4A12, the antibody reduces or inhibits activity of MS4A12.

Preferably, the antibody binds to an extracellular region of MS4A12, i.e., a region of MS4A12 which is extracellularly accessible when MS4A12 is expressed by a cell. "Extracellularly accessible" in this respect means that an agent such as an antibody can bind to an extracellular target when added to the extracellular environment of a cell, preferably without having to enter into or cross the plasma membrane of said cell. Preferably, the antibody binds to a region of human MS4A12 within amino acid numbers 113 to 126 or 182 to 201 of SEQ ID NO: 2 or a corresponding region in a derivative, isoform, variant or homolog of human MS4A12.

The antibody of the invention may be monoclonal or polyclonal, preferably monoclonal. Furthermore, the antibody of the invention may be a chimeric or humanized antibody or a fragment of an antibody such as the F(ab')₂, Fab, Fv or Fd fragment of an antibody.

In another embodiment, the agent which reduces or inhibits expression or activity of MS4A12 of the invention is a nucleic acid molecule, preferably a recombinant nucleic acid molecule, capable of expressing the siRNA, antisense nucleic acid or antibody of the invention. Preferably the nucleic acid molecule comprises a promoter functionally linked to a nucleic acid sequence for expressing the siRNA, antisense nucleic acid or antibody of the invention, preferably an inducible or regulatable promoter. Preferably, the nucleic acid molecule is a vector such as an expression plasmid or a viral vector. The viral vector may be selected from the group consisting of an adenoviral vector, an adeno-associated viral vector, a lentiviral vector, a retroviral vector, and a herpes virus vector.

The nucleic acid molecule capable of expressing the siRNA may be a single vector comprising nucleic acid sequences for expressing the sense RNA strand and the antisense RNA strand of the siRNA or a combination of two vectors, one vector comprising a nucleic acid sequence for expressing the sense RNA strand and one vector comprising a nucleic acid sequence for expressing the antisense RNA strand of the siRNA.

In one embodiment, the nucleic acid molecule is present in a host cell. Preferably, the nucleic acid molecule present in the host cell is capable of expressing the antibody of the invention. In a preferred embodiment, the antibody expressed by the nucleic acid molecule is secreted by the host cell.

In another embodiment, the agent which reduces or inhibits expression or activity of MS4A12 of the invention comprises, and preferably consists of a compound selected from the group consisting of a peptide, a protein, a nucleic acid, and a small molecule compound, which is capable of reducing or inhibiting expression or activity of MS4A12.

For example, peptides, proteins, nucleic acids and small molecule compounds capable of reducing or inhibiting expression or activity of MS4A12 may be identified by any suitable screening assay known in the art. A library of different peptides, proteins, nucleic acids and/or small molecule compounds may be used in the screening assay. The library may be composed of chemically synthesized compounds, naturally occurring compounds or compounds derived from naturally occurring compounds. Suitable libraries are known in the art. For example, a peptide or protein library may be a phage display library comprising phages displaying the peptide or protein on their surface. The screening assay may comprise one or more rounds of selection comprising one or more selection steps. The members of the library may be screened for binding to MS4A12, reducing or inhibiting expression of MS4A12, and/or reducing or inhibiting activity of MS4A12.

In a further embodiment, the agent which reduces or inhibits expression or activity of MS4A12 of the invention is a mixture of different agents. In particular, the agent may be a mixture of two or more siRNAs as defined above, a mixture of two or more antisense nucleic acids as defined above, or a mixture of two or more antibodies as defined above. Furthermore, the agent may be a mixture of one or more siRNAs as defined above, and/or one or more antisense nucleic acids as defined above, and/or one or more antibodies as defined above.

In particular embodiments, the MS4A12 mRNA or MS4A12 gene comprises a nucleic acid sequence which is selected from the group consisting of (a) the nucleic acid sequence of SEQ ID NO: 1, a part or derivative thereof, (b) a nucleic acid sequence which hybridizes with the nucleic acid sequence of (a) under stringent conditions, (c) a nucleic acid sequence which is degenerate with respect to the nucleic acid of (a) or (b), (d) a nucleic acid sequence which is complementary to the nucleic acid sequence of (a), (b) or (c), and (e) a nucleic acid sequence which encodes the amino acid sequence of SEQ ID NO: 2, a part, variant, isoform or homolog thereof.

Furthermore, in particular embodiments the MS4A12 protein comprises an amino acid sequence which is selected from the group consisting of the amino acid sequence of SEQ ID NO: 2, a part, variant, isoform or homolog thereof.

The agent which reduces or inhibits expression or activity of MS4A12 of the invention is useful for treating diseases wherein a reduction or inhibition of MS4A12 expression or activity is beneficial for treatment, in particular cancer and cancer metastasis, preferably colon cancer and colon cancer metastasis. In particular, the agent which reduces or inhibits expression or activity of MS4A12 of the invention is capable of reducing or inhibiting tumor growth, tumor invasion and/or tumor metastasis. In one embodiment, the agent which reduces or inhibits expression or activity of MS4A12 of the invention is capable of reducing or inhibiting store-operated Ca²⁺ entry in a cell.

The invention furthermore relates to a composition which comprises the agent which reduces or inhibits expression or activity of MS4A12 of the invention. Preferably, the composition comprises the agent which reduces or inhibits expression or activity of MS4A12 of the invention in an amount and/or concentration which is effective to reduce or inhibit expression or activity of MS4A12 in a cell. The composition may comprise only one agent which reduces or inhibits expression or activity of MS4A12 of the invention or a combination of different agents which reduce or inhibit expression or activity of MS4A12 of the invention.

In one embodiment, the composition of the invention further comprises an agent which reduces or inhibits expression or activity of an EGF receptor in a cell.

An "agent which reduces or inhibits expression of an EGF receptor in a cell" may reduce or inhibit transcription of the EGF receptor gene, reduce or inhibit procession of the EGF receptor pre-mRNA (such as splicing, poly-adenylation, capping, etc.), reduce the half-life of the EGF receptor mRNA, and/or reduce or inhibit translation of the EGF receptor mRNA into protein. In particular, the amount of EGF receptor protein synthesized from an EGF receptor gene in a cell is reduced in the presence of the agent which reduces or inhibits expression of an EGF receptor in a cell.

An "agent which reduces or inhibits activity of an EGF receptor in a cell" may reduce or inhibit one or more activities of an EGF receptor. The term "activity of an EGF receptor" refers to any response which a cell bearing an EGF receptor exhibits upon activation of the EGF receptor, in particular by binding of EGF to the EGF receptor. However, as further explained below, activity of an EGF receptor in a cell according to the invention may be reduced or inhibited not only by interfering with the EGF receptor as such, but any component involved in EGF signaling.

Preferably, the activity of an EGF receptor is involved in formation, progression and/or maintenance of tumors or tumor metastases such as colon tumors or colon tumor metastases. Preferably, the activity of an EGF receptor is selected from the group consisting of increasing or enabling cell growth, proliferation, viability, cell cycle progression, migration, chemotaxis and invasion.

The agent which reduces or inhibits activity of an EGF receptor in a cell may reduce or inhibit said activity by directly interacting with the EGF receptor. For example, the agent may block the ligand binding site of the EGF receptor and thereby prevent EGF from binding to the receptor. An antibody specific for the EGF receptor, an antagonistic EGF mimetic or a small molecule compound capable of binding to the ligand binding site may be suitable in this respect. Furthermore, the agent may block the dimerization of the EGF receptor. Examples of such an agent are inactive variants of an EGF receptor monomer, in particular dominant negative variants thereof, and antibodies or small molecule compounds binding to the dimerization interaction surface of the EGF receptor monomer. Moreover, the agent may block the protein tyrosine kinase domain of the EGF receptor, i.e., the agent may be a tyrosine kinase inhibitor, preferably a small molecule tyrosine kinase inhibitor. Furthermore, the agent may block the interaction of the EGF receptor with its downstream interaction partners, e.g. PLCγ, for example by binding to the binding site of the EGF receptor for binding said interaction partners.

In another embodiment, the agent which reduces or inhibits activity of an EGF receptor in a cell does not directly interact with the EGF receptor. In particular, the agent may bind to EGF and thus prevent EGF from binding to the receptor, prevent production or secretion of EGF, or degrade EGF. Antibodies specific for EGF are an example for such an agent. Furthermore, the agent may reduce or inhibit one or more, preferably all signal transduction pathways induced by the EGF receptor, in particular the signal transduction pathway leading to intracellular Ca²⁺ store depletion and/or store-operated Ca²⁺ entry. For example, the agent may reduce or inhibit expression or activity of one or more proteins involved in said signal transduction pathways, for example, PLCγ. Examples of such agents are siRNAs and antisense nucleic acids reducing or inhibiting expression of such proteins, and antibodies or small molecule compounds reducing or inhibiting one or more activities of such proteins, for example, an enzymatic activity or an ability to bind to an interaction partner.

It is to be understood that reduction or inhibition of expression of an EGF receptor in a cell preferably will also result in reduction or inhibition of activity of the EGF receptor in said cell as discussed above.

In a preferred embodiment, the agent which reduces or inhibits expression or activity of an EGF receptor reduces or inhibits expression or activity of more than one different EGF receptor, preferably of all different EGF receptors present in a cell. The EGF receptor is preferably a human EGF receptor such as human EGFR, human ErbB-1, or human HER1. However, the EGF receptor may also be a species homolog of these human EGF receptors.

Preferably, the agent which reduces or inhibits expression or activity of an EGF receptor in a cell is selected from the group consisting of (i) an siRNA specifically targeting an EGF receptor mRNA; (ii) an antisense nucleic acid capable of hybridizing selectively with a nucleic acid coding for an EGF receptor; (iii) an antibody capable of selectively binding to an EGF receptor; (iv) a nucleic acid molecule capable of expressing the siRNA of (i), the antisense nucleic acid of (ii) or the antibody of (iii); and (v) a tyrosine kinase inhibitor specific for an EGF receptor.

Particular examples of an agent which reduces or inhibits expression or activity of an EGF receptor in a cell are the antibodies cetuximab, panitumumab, zalutumumab, nimotuzumab and matuzumab and the tyrosine kinase inhibitors gefitinib, erlotinib and lapatinib (e.g. Sridhar et al., Lancet Oncol. 4: 397-406, 2003).

In one embodiment, the composition of the invention is a pharmaceutical composition. The pharmaceutical composition of the invention is preferably useful for treating diseases wherein a reduction or inhibition of MS4A12 expression or activity and/or a reduction or inhibition of EGF receptor expression or activity is beneficial for treatment, in particular cancer and cancer metastasis, preferably colon cancer and colon cancer metastasis. A pharmaceutical composition of the invention may comprise a pharmaceutically compatible carrier.

The invention furthermore relates to a method for reducing or inhibiting responsiveness of a cell towards EGF comprising contacting said cell with an agent which reduces or inhibits expression or activity of MS4A12 of the invention. Preferably, the cell is contacted with the agent which reduces or inhibits expression or activity of MS4A12 of the invention in an amount and/or concentration which is effective to reduce or inhibit expression or activity of MS4A12 in said cell.

In one embodiment, the contacting of the cell is achieved by adding the agent to the extracellular environment of the cell. In this embodiment, the agent preferably is an antibody as described above. In another embodiment, contacting the cell with the agent includes introducing the agent into the cell. In this embodiment, the agent preferably is an siRNA, an antisense nucleic acid or a nucleic acid molecule capable of expressing an siRNA or an antisense nucleic acid, as described above. Introduction of the agent into the cell may be achieved by any method known in the art. For example, direct injection, introduction using a lipid vehicle such as a liposome, electroporation, or velocity-driven transfection methods (e.g. gene gun) may be used for the introduction of any agent which reduces or inhibits expression or activity of MS4A12 of the invention into the cell. Furthermore, transfection using a vector such as a plasmid vector or viral vector, calcium phosphate precipitation, or binding to cationic polymers such as DEAE-dextran or polyethylenimine may be used for the introduction of an agent which reduces or inhibits expression or activity of MS4A12 of the invention which is composed of nucleic acids such as siRNA, antigen nucleic acid and nucleic acid molecule capable of expressing an siRNA, antigen nucleic acid or antibody.

Preferably, the cell responsive to EGF expresses MS4A12 and more preferably additionally expresses an EGF receptor. Preferably, the cell is a neoplastic cell such as a tumor cell.

In one embodiment, the method for reducing or inhibiting responsiveness of a cell towards EGF further comprises contacting said cell with an agent which reduces or inhibits expression or activity of an EGF receptor in a cell. The agent which reduces or inhibits expression or activity of an EGF receptor in a cell preferably is an agent as described above. In particular, said agent is preferably selected from the group consisting of (i) an siRNA specifically targeting an EGF receptor mRNA; (ii) an antisense nucleic acid capable of hybridizing selectively with a nucleic acid coding for an EGF receptor; (iii) an antibody capable of selectively binding to an EGF receptor; (iv) a nucleic acid molecule capable of expressing the siRNA of (i), the antisense nucleic acid of (ii) or the antibody of (iii); and (v) a tyrosine kinase inhibitor specific for an EGF receptor.

Contacting the cell with the agent which reduces or inhibits expression or activity of an EGF receptor in a cell may be achieved in a similar manner as described above for the agent which reduces or inhibits expression or activity of MS4A12 of the invention. The cell may be contacted with the agent which reduces or inhibits expression or activity of an EGF receptor in a cell and the agent which reduces or inhibits expression or activity of MS4A12 of the invention simultaneously or consecutively. If the cell is simultaneously contacted with both agents, the agents may be present in one composition or in separate compositions.

In one embodiment, the method for reducing or inhibiting responsiveness of a cell towards EGF is performed in vitro. In this embodiment, the cell may be an isolated cell, preferably a cell cultured *in vitro.* Furthermore, the cell may be comprised in a tissue sample which preferably is isolated from an organism.

In another embodiment, the method for reducing or inhibiting responsiveness of a cell towards EGF is performed in vivo. In this embodiment, the cell is present in an organism such as an animal, preferably a mammal such as a rodent like mouse or rat, a primate or a human being. Furthermore, in this embodiment, contacting the cell with the agent which reduces or inhibits expression or activity of MS4A12 of the invention and optionally the agent which reduces or inhibits expression or activity of an EGF receptor in a cell preferably comprises administering said agent(s) to the organism. Administration of the agent(s) may be performed as described below and/or may comprise any of the methods for introducing the agent(s) into a cell described above. Preferably, for administration, the agent(s) is/are present in a pharmaceutical composition as described herein.

Preferably, if the method for reducing or inhibiting responsiveness of a cell towards EGF is performed in vivo it is performed to treat cancer and/or cancer metastasis.

Thus, in a further aspect, the invention relates to a method of treating cancer and/or cancer metastasis, in particular colon cancer and/or colon cancer metastasis. The treatment comprises administering the agent which reduces or inhibits expression or activity of MS4A12 of the invention to a patient. In particular, the method comprises administering an effective amount of the agent which reduces or inhibits expression or activity of MS4A12 of the invention to a patient in need thereof, preferably a patient having colon cancer or colon cancer metastasis. Preferably, tumor growth, tumor invasion and/or tumor metastasis are reduced or inhibited in the method.

In one embodiment, the method of treating cancer and/or cancer metastasis further comprises administering to the patient an agent which reduces or inhibits expression or activity of an EGF receptor in a cell. The agent which reduces or inhibits expression or activity of an EGF receptor in a cell preferably is an agent as described above. In particular, said agent is preferably selected from the group consisting of (i) an siRNA specifically targeting an EGF receptor mRNA; (ii) an antisense nucleic acid capable of hybridizing selectively with a nucleic acid coding for an EGF receptor; (iii) an antibody capable of selectively binding to an EGF receptor; (iv) a nucleic acid molecule capable of expressing the siRNA of (i), the antisense nucleic acid of (ii) or the antibody of (iii); and (v) a tyrosine kinase inhibitor specific for an EGF receptor. Preferably, the agent which reduces or inhibits expression or activity of an EGF receptor in a cell is administered in an effective amount.

Preferably, the agent which reduces or inhibits expression or activity of MS4A12 of the invention and optionally the agent which reduces or inhibits expression or activity of an EGF receptor in a cell are administered in a pharmaceutical composition as described herein. The agent which reduces or inhibits expression or activity of an EGF receptor in a cell and the agent which reduces or inhibits expression or activity of MS4A12 of the invention may be administered simultaneously or consecutively. If both agents are administered simultaneously, they may be present in one pharmaceutical composition or in separate pharmaceutical compositions.

According to the invention, the terms "cancer" and "cancer metastasis" preferably relate to cancer or cancer metastasis, respectively, which are characterized by expression of MS4A12 and/or expression of an EGF receptor, i.e., at least a part of the cancer cells of the cancer or cancer metastasis express MS4A12 and/or at least a part of the cancer cells express an EGF receptor. In a preferred embodiment, at least a part of the cancer cells express and/or secrete EGF.

Preferably, at least a part of the cancer cells of the cancer or cancer metastasis are responsive towards EGF. Preferably, in the cells responsive towards EGF, cell growth, proliferation, viability, cell cycle progression, migration, chemotaxis and/or invasion are increased in the presence of EGF. In one embodiment, tumor growth, tumor invasion and/or tumor metastasis in the cancer or cancer metastasis are increased in the presence of EGF.

The invention furthermore relates to a method for determining the responsiveness of a cell towards EGF comprising determining the level of expression or activity of MS4A12 in the cell. This method preferably allows to draw qualitative and/or quantitative conclusions on whether and to what extent a cell will respond towards an addition of EGF.

In one embodiment, the determination of the level of expression of MS4A12 in the cell comprises determining the amount of MS4A12 mRNA and/or MS4A12 protein in said cell. Preferably, determination of the amount of MS4A12 mRNA and/or MS4A12 protein comprises contacting the cell with an agent which binds specifically to the MS4A12 mRNA or MS4A12 protein, and determining the amount of a complex between the agent and the MS4A12 mRNA or MS4A12 protein.

According to the invention, determination of the amount of MS4A12 mRNA may be carried out using an oligo- or polynucleotide probe which hybridizes specifically to said MS4A12 mRNA or may be carried out by selective amplification of said MS4A12 mRNA, e.g. by means of PCR amplification. In one embodiment, the oligo- or polynucleotide probe comprises a sequence which is complementary to 6-50, in particular 10-30, 15-30 and 20-30, contiguous nucleotides of said MS4A12 mRNA.

According to the invention, determination of the amount of MS4A12 protein may be carried out using an antibody binding specifically to said MS4A12 protein.

In the method for determining the responsiveness of a cell towards EGF, the agent which is used for determining the amount of MS4A12 mRNA and/or MS4A12 protein is preferably labeled in a detectable manner, in particular by a detectable marker such as a radioactive marker, a fluorescent marker or an enzymatic marker.

Preferably, presence of MS4A12 mRNA or MS4A12 protein in the cell indicates that the cell is responsive towards EGF. Preferably, the amount of MS4A12 mRNA or MS4A12 protein in the cell correlates with the expected level of responsiveness of said cell towards EGF. Thus, the higher the amount of MS4A12 mRNA or MS4A12 protein in the cell, the higher the expected level of responsiveness of said cell towards EGF.

Determining the level of activity of MS4A12 in the cell may include determining one or more activities of MS4A12 in the cell.

In preferred embodiments, the method for determining the responsiveness of a cell towards EGF comprises determining the level of activity of MS4A12 in the cell in the presence and/or in the absence of an agent which reduces or inhibits expression or activity of MS4A12, in particular an agent which reduces or inhibits expression or activity of MS4A12 of the invention.

Preferably, a detectable level of activity of MS4A12 is indicative for a responsiveness of the cell towards EGF. Preferably, the level of activity of MS4A12 correlates with the expected level of responsiveness of the cell towards EGF.

If the level of activity of MS4A12 is determined in the presence and/or in the absence of an agent which reduces or inhibits expression or activity of MS4A12, a decrease in the level of activity of MS4A12 in the presence of said agent is indicative for a responsiveness of the cell towards EGF. The extent of said decrease in the level of activity of MS4A12 in the presence of said agent preferably correlates with the expected level of responsiveness of the cell towards EGF.

In one embodiment, determining the level of activity of MS4A12 in the cell comprises determining store-operated Ca²⁺ entry in said cell. Preferably, the store-operated Ca²⁺ entry is determined in the presence and absence of an agent capable of reducing or inhibiting expression or activity of MS4A12, in particular an agent which reduces or inhibits expression or activity of MS4A12 of the invention. Preferably, said agent is selected from the group consisting of (i) an siRNA specifically targeting MS4A12 mRNA; (ii) an antisense nucleic acid capable of hybridizing selectively with a nucleic acid coding for MS4A12; (iii) an antibody capable of selectively binding to MS4A12; and (iv) a nucleic acid molecule capable of expressing the siRNA of (i) or the antisense nucleic acid of (ii).

In one embodiment, the determination of store-operated Ca²⁺ entry in the cell comprises determining the amount and/or time course of Ca²⁺ and/or Sr²⁺ entry into the cell.

Determining the amount of Ca²⁺ and/or Sr²⁺ entry into a cell preferably comprises determining the intracellular Ca²⁺ and/or Sr²⁺ concentrations at the beginning of the measurement and determining the intracellular Ca²⁺ and/or Sr²⁺ concentrations at the end of the measurement and calculating the difference between the Ca²⁺ and/or Sr²⁺ concentrations at the end of the measurement and the Ca²⁺ and/or Sr²⁺ concentrations at the beginning of the measurement, wherein said difference corresponds to the amount of Ca²⁺ and/or Sr²⁺ entry into the cell.

Determining the time course of Ca²⁺ and/or Sr²⁺ entry into a cell preferably comprises monitoring the intracellular Ca²⁺ and/or Sr²⁺ concentrations for a certain period of time, wherein an increase of the Ca²⁺ and/or Sr²⁺ concentrations is indicative for an entry of Ca²⁺ and/or Sr²⁺, while a decrease is indicative for an efflux of Ca²⁺ and/or Sr²⁺. From the time course of Ca²⁺ and/or Sr²⁺ entry into the cell one can calculate the amount of Ca²⁺ and/or Sr²⁺ entering the cell during a given period of time as well as the velocity of Ca²⁺ and/or Sr²⁺ entry.

In one embodiment, the amount and/or time course of Ca²⁺ and/or Sr²⁺ entry into the cell is determined at a given extracellular Ca²⁺ and/or Sr²⁺ concentration. Extracellular Ca²⁺ and/or Sr²⁺ may be provided by adding CaCl₂ and/or SrCl₂ to the extracellular environment. The given extracellular Ca²⁺ and/or Sr²⁺ concentration preferably is about 0.01 mM to about 100 mM, more preferably about 0.1 mM to about 10 mM, more preferably about 0.5 mM to about 2 mM, and most preferably about 1 mM.

Ca²⁺ and/or Sr²⁺ entry may be measured by a compound sensitive to the presence of Ca²⁺ and/or Sr²⁺. Preferably, said compound provides a detectable signal which changes in the absence and presence of Ca²⁺ and/or Sr²⁺. Preferably, the detectable signal changes with and more preferably can be correlated with the concentration of Ca²⁺ and/or Sr²⁺. In one embodiment, the compound sensitive to the presence of Ca²⁺ and/or Sr²⁺ is a Ca²⁺-sensitive dye. In another embodiment, the compound is labeled with a detectable marker wherein the marker provides the detectable signal. The detectable marker preferably is a fluorescent marker or an enzymatic marker.

In a preferred embodiment, the compound which is sensitive to the presence of Ca²⁺ and/or Sr²⁺ is present in the cell to be assayed, more preferably only in the cytoplasm of the cell. For example, the FLIPR Calcium 3 Assay Kit (Molecular Devices) may be used according to the manufacturer's instructions. Store-operated Ca²⁺ entry may be measured using a microscope.

In one embodiment, the amount and/or time course of Ca²⁺ and/or Sr²⁺ entry into a cell is determined after depletion of an intracellular Ca²⁺ store. Said depletion may be achieved by any means known in the art, for example by administration of an inhibitor of the endoplasmatic reticular Ca²⁺-ATPase (SERCA) such as thapsigargin or a natural activator of Ca²⁺ store depletion such as EGF. Thus, in one embodiment, the method further comprises contacting the cell with EGF and/or thapsigargin. Preferably, intracellular Ca²⁺ stores are depleted in the absence of extracellular Ca²⁺ and/or Sr²⁺ and Ca²⁺ and/or Sr²⁺ is added to the extracellular environment of the cell following said depletion.

In the determination of store-operated Ca²⁺ entry, Sr²⁺ may be used as substitute for Ca²⁺ and the amount and/or time course of Sr²⁺ entry may be taken as measure for the amount and/or time course of Ca²⁺ entry.

Preferably, detectable store-operated Ca²⁺ entry is indicative for a responsiveness of the cell towards EGF. Preferably, the extent of store-operated Ca²⁺ entry corresponds with the expected level of responsiveness of the cell towards EGF.

If the store-operated Ca²⁺ entry is determined in the presence and in the absence of an agent which reduces or inhibits expression or activity of MS4A12, a decrease in store-operated Ca²⁺ entry in the presence of said agent is indicative for a responsiveness of the cell towards EGF. The extent of said decrease in store-operated Ca²⁺ entry in the presence of said agent preferably correlates with the expected level of responsiveness of the cell towards EGF.

In one embodiment of the method for determining the responsiveness of a cell towards EGF, the results of the determination of the level of expression or activity of MS4A12 in the cell are compared to the results of the determination of the level of expression or activity of MS4A12 in a reference cell. Preferably, the responsiveness of the reference cell towards EGF is known. In one embodiment, the reference cell is a tumor cell. Preferably, the cell which is to be analyzed and the reference cell are of the same species and/or of the same type.

In certain embodiments, the method for determining the responsiveness of a cell towards EGF further comprises determining the level of expression or activity of an EGF receptor in the cell. Preferably, the level of expression of an EGF receptor is determined by determining the amount of EGF receptor mRNA and/or EGF receptor protein in the cell using, for example, an agent specifically binding to EGF receptor mRNA such as a nucleic acid molecule specifically hybridizing with EGF receptor mRNA, and/or an agent specifically binding to EGF receptor protein such as an antibody specific for EGF receptor protein. The level of activity of an EGF receptor may be determined by determining the enzymatic activity of the EGF receptor, in particular its activity to phosphorylate a target protein.

In one embodiment of the method for determining the responsiveness of a cell towards EGF, the cell is a eukaryotic cell, preferably a mammalian cell, more preferably a human cell. Preferably, the cell is a tumor cell. In one embodiment, the responsiveness towards EGF of more than one cell is determined simultaneously.

Preferably, the method for determining the responsiveness of a cell towards EGF is performed in vitro. In one embodiment, the cell is a tumor cell derived from or present in a tumor sample. In this embodiment, the method is useful for determining the responsiveness towards EGF of the tumor from which the tumor cell is derived.

The tumor sample may be any sample containing tumor cells, including disseminating tumor cells or metastatic tumor cells. Preferably, the tumor sample is a sample of a colon tumor or colon tumor metastasis and/or the tumor cells are cells of a colon tumor or a metastasis of colon tumor origin. The tumor sample may be a sample from a biopsy of a tumor or a tissue containing metastatic tumor cells. Furthermore, the tumor sample may be a biological sample such as feces. Preferably, the tumor sample is obtained from a patient being diagnosed to have cancer, preferably colon cancer.

Responsiveness of a tumor towards EGF implies that the tumor is treatable by the method of treatment of the invention and/or another anti-EGF tumor therapy, in particular a tumor therapy including the administration of an agent which reduce or inhibit expression or activity of an EGF receptor as described herein. Thus, if a tumor is determined by the method of the invention as being responsive towards EGF, it may be expected that the patient having said tumor may successfully be treated by the method of treatment of the invention and/or another anti-EGF tumor therapy.

The invention further relates to a method for increasing responsiveness of a cell towards EGF. The method comprises increasing expression and/or activity of MS4A12 in said cell.

In one embodiment, the method comprises introducing MS4A12 protein or a nucleic acid molecule capable of expressing MS4A12 protein into the cell. Preferably, the nucleic acid molecule is a vector such as an expression plasmid or a viral vector. Introduction of the MS4A12 protein or the nucleic acid molecule capable of expressing MS4A12 protein into the cell may be achieved by any method known in the art. For example, direct injection, introduction using a lipid vehicle such as a liposome, electroporation, or velocity-driven transfection methods (e.g. gene gun) may be used. The nucleic acid molecule capable of expressing MS4A12 protein may also be introduced into the cell by transfection using, for example, viral transfection, calcium phosphate precipitation or binding to cationic polymers such as DEAE-dextran or polyethylenimine.

Preferably, the cell expresses an EGF receptor. In one embodiment, the method for increasing responsiveness of a cell towards EGF further comprises increasing expression and/or activity of an EGF receptor in said cell. In this embodiment, the method preferably comprises introducing an EGF receptor or a nucleic acid molecule capable of expressing an EGF receptor into the cell. Introduction thereof may be achieved as described above for the introduction of MS4A12 protein or a nucleic acid molecule capable of expressing MS4A12 protein.

The method for increasing responsiveness of a cell towards EGF may be performed in vitro as well as in vivo.

### Detailed description of the invention

A reference herein to a range of numerical values is to be understood so as to specify and mention each of the individual numerical values comprised by said range.

The term "MS4A12" relates to "membrane-spanning 4-domains, subfamily A, member 12" and includes any variants, in particular splice variant, conformations, isoforms and species homologs of MS4A12 which are naturally expressed by cells or are expressed by cells transfected with the MS4A12 gene.

Preferably, a "nucleic acid of MS4A12", a "nucleic acid encoding MS4A12", a "nucleic acid coding for MS4A12" or "MS4A12 gene" relates to a nucleic acid selected from the group consisting of (a) the nucleic acid sequence of SEQ ID NO: 1, a part or derivative thereof, (b) a nucleic acid sequence which hybridizes with the nucleic acid sequence of (a) under stringent conditions, (c) a nucleic acid sequence which is degenerate with respect to the nucleic acid of (a) or (b), and (d) a nucleic acid sequence which is complementary to the nucleic acid sequence of (a), (b) or (c). The terms may also include mRNA coding for MS4A12.

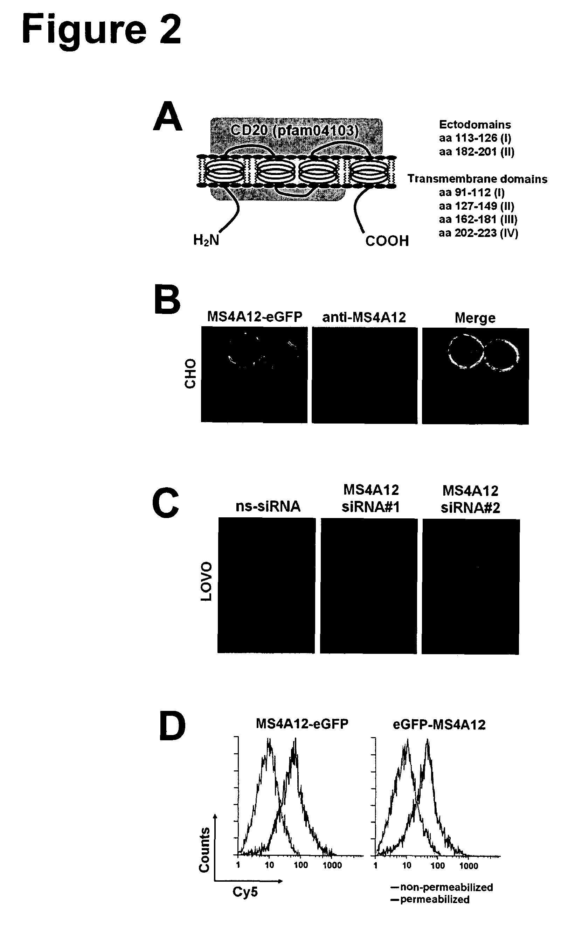

Preferably, "MS4A12 protein" or simply "MS4A12" comprises an amino acid sequence encoded by the aforementioned nucleic acid, preferably an amino acid sequence selected from the group consisting of SEQ ID NOs: 2, a part or derivative thereof. When expressed in a cell, the MS4A12 protein preferably is located at the plasma membrane, more preferably incorporated into the plasma membrane. In preferred embodiments, MS4A12 is incorporated into the plasma membrane of a cell in such a manner that its amino- and carboxy-termini are located in the cytoplasm, and one additional cytoplasmic domain, two extracellular domains and four transmembrane domains are present. Preferably, the extracellular domains of MS4A12 correspond to the amino acid numbers 113 to 126 and 182 to 201 of SEQ ID NO: 2 or the corresponding amino acid numbers in derivatives, isoforms or species homologs of human MS4A12. It is to be understood that the boundaries of the extracellular domains of MS4A12 may also be shifted by 1, 2, 3, 4, or 5 amino acid positions in the amino acid sequence of MS4A12. MS4A12 preferably has a plasma membrane topology as shown in Fig. 2A.

The term "MS4A12" also includes posttranslationally modified variants, isoforms and species homologs of human MS4A12 which are naturally expressed by cells or are expressed by cells transfected with the MS4A12 gene.

According to the invention, a nucleic acid is preferably deoxyribonucleic acid (DNA) or ribonucleic acid (RNA). Nucleic acids comprise according to the invention genomic DNA, cDNA, mRNA, recombinantly produced and chemically synthesized molecules. According to the invention, a nucleic acid may be present as a single-stranded or double-stranded and linear or covalently circularly closed molecule.

As used herein, the term "RNA" means a molecule comprising at least one ribonucleotide residue. By "ribonucleotide" is meant a nucleotide with a hydroxyl group at the 2'-position of a beta-D-ribo-furanose moiety. The term includes double stranded RNA, single stranded RNA, isolated RNA such as partially purified RNA, essentially pure RNA, synthetic RNA, recombinantly produced RNA, as well as altered RNA that differs from naturally occurring RNA by the addition, deletion, substitution and/or alteration of one or more nucleotides. Such alterations can include addition of non-nucleotide material, such as to the end(s) of an RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA.

If reference is made herein to the detection of or the determination of the quantity of a nucleic acid, the nucleic acid which is actually to be detected or the quantity of which is actually to be determined is preferably mRNA. However, it should be understood that this may also include embodiments wherein mRNA is detected or the quantity of mRNA is determined indirectly. For example, mRNA may be transformed into cDNA and the cDNA detected or its quantity determined. mRNA is given herein as the cDNA equivalent. One skilled in the art would understand that the cDNA sequence is equivalent to the mRNA sequence, and can be used for the same purpose herein, e.g., the generation of probes hybridizing to the nucleic acid to be detected. Thus, if reference is made herein to the sequences shown in the sequence listing this is also to include the RNA equivalents of said sequences.

The nucleic acids described according to the invention have preferably been isolated. The term "isolated nucleic acid" means according to the invention that the nucleic acid was (i) amplified *in vitro,* for example by polymerase chain reaction (PCR), (ii) recombinantly produced by cloning, (iii) purified, for example by cleavage and gel-electrophoretic fractionation, or (iv) synthesized, for example by chemical synthesis. An isolated nucleic acid is a nucleic acid which is available for manipulation by recombinant DNA techniques.

A degenerate nucleic acid according to the invention is a nucleic acid that differs from a reference nucleic acid in codon sequence due to the degeneracy of the genetic code.

"Derivative" of a nucleic acid means according to the invention that single or multiple such as at least 2, at least 4, or at least 6 and preferably up to 3, up to 4, up to 5, up to 6, up to 10, up to 15, or up to 20 nucleotide substitutions, deletions and/or additions are present in said nucleic acid. Furthermore, the term "derivative" also comprises chemical derivatization of a nucleic acid on a nucleotide base, on the sugar or on the phosphate. The term "derivative" also comprises nucleic acids which contain nucleotides and nucleotide analogs not occurring naturally. Furthermore, the term "derivative" with respect to RNA also comprises RNA which has been modified by stabilizing sequences, capping and/or polyadenylation.

Preferably the degree of identity between a specific nucleic acid sequence described herein and a nucleic acid sequence which is a derivative of said specific nucleic acid sequence, which hybridizes with said specific nucleic acid sequence and/or which is degenerate with respect to said specific nucleic acid sequence will be at least 70%, preferably at least 75%, preferably at least 80%, more preferably at least 85%, even more preferably at least 90% or most preferably at least 95%, 96%, 97%, 98% or 99%. The degree of identity is preferably given for a region of at least about 30, at least about 50, at least about 70, at least about 90, at least about 100, at least about 150, at least about 200, at least about 250, at least about 300, or at least about 400 nucleotides. In preferred embodiments, the degree of identity is given for the entire length of the reference nucleic acid sequence, such as the nucleic acid sequences given in the sequence listing.

A nucleic acid is "complementary" to another nucleic acid if the two sequences are capable of hybridizing and forming a stable duplex with one another, with hybridization preferably being carried out under conditions which allow specific hybridization between polynucleotides (stringent conditions). Stringent conditions are described, for example, in Molecular Cloning: A Laboratory Manual, J. Sambrook et al., Editors, 2nd Edition, Cold Spring Harbor Laboratory press, Cold Spring Harbor, New York, 1989 or Current Protocols in Molecular Biology, F.M. Ausubel et al., Editors, John Wiley & Sons, Inc., New York and refer, for example, to hybridization at 65°C in hybridization buffer (3.5 x SSC, 0.02% Ficoll, 0.02% polyvinylpyrrolidone, 0.02% bovine serum albumin, 2.5 mM NaH₂PO₄ (pH 7), 0.5% SDS, 2 mM EDTA). SSC is 0.15 M sodium chloride/0.15 M sodium citrate, pH 7. After hybridization, the membrane to which the DNA has been transferred is washed, for example, in 2 x SSC at room temperature and then in 0.1-0.5 x SSC/0.1 x SDS at temperatures of up to 68°C.

A percent complementarity indicates the percentage of contiguous residues in a nucleic acid molecule that can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" or "fully complementary" means that all the contiguous residues of a nucleic acid sequence will hydrogen bond with the same number of contiguous residues in a second nucleic acid sequence. Preferably, the degree of complementarity according to the invention is at least 70%, preferably at least 75%, preferably at least 80%, more preferably at least 85%, even more preferably at least 90% or most preferably at least 95%, 96%, 97%, 98% or 99%. Most preferably, the degree of complementarity according to the invention is 100%.

"Sequence similarity" indicates the percentage of amino acids that either are identical or that represent conservative amino acid substitutions. "Sequence identity" between two polypeptide or nucleic acid sequences indicates the percentage of amino acids or nucleotides that are identical between the sequences.

The term "percentage identity" is intended to denote a percentage of nucleotides or of amino acid residues which are identical between the two sequences to be compared, obtained after the best alignment, this percentage being purely statistical and the differences between the two sequences being distributed randomly and over their entire length. Sequence comparisons between two nucleotide or amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl. Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

Nucleic acids coding for a peptide or protein or nucleic acids capable of expressing an siRNA or antisense nucleic acid may, according to the invention, be present alone or in combination with other nucleic acids, in particular heterologous nucleic acids. In preferred embodiments, a nucleic acid is functionally linked to expression control sequences or regulatory sequences which may be homologous or heterologous with respect to said nucleic acid. A coding/expression sequence and a regulatory sequence are "functionally" linked to one another, if they are covalently linked to one another in such a way that expression or transcription of said coding/expression sequence is under the control or under the influence of said regulatory sequence. If the coding sequence is to be translated into a functional protein, then, with a regulatory sequence functionally linked to said coding sequence, induction of said regulatory sequence results in transcription of said coding sequence, without causing a frame shift in the coding sequence or said coding sequence not being capable of being translated into the desired protein or peptide.

The term "expression control sequence" or "regulatory sequence" comprises, according to the invention promoters, enhancers and other control elements which regulate expression of a gene. In particular embodiments of the invention, the expression control sequences can be regulated. The exact structure of regulatory sequences may vary as a function of the species or cell type, but generally comprises 5'-untranscribed and 5'-untranslated sequences which are involved in initiation of transcription and translation, respectively, such as TATA box, capping sequence, CAAT sequence, and the like. More specifically, 5'-untranscribed regulatory sequences comprise a promoter region which includes a promoter sequence for transcriptional control of the functionally linked gene. Regulatory sequences may also comprise enhancer sequences or upstream activator sequences.

According to the invention, a nucleic acid may furthermore be present in combination with another nucleic acid which codes for a peptide controlling secretion of the protein or peptide encoded by said nucleic acid from a host cell. According to the invention, a nucleic acid may also be present in combination with another nucleic acid which codes for a peptide causing the encoded protein or peptide to be anchored on the cell membrane of the host cell or compartmentalized into particular organelles of said cell. Similarly, a combination with a nucleic acid is possible which represents a reporter gene or any "tag".

In a preferred embodiment, a recombinant nucleic acid molecule is according to the invention a vector, where appropriate with a promoter, which controls expression of a nucleic acid. The term "vector" is used here in its most general meaning and comprises any intermediary vehicle for a nucleic acid which enables said nucleic acid, for example, to be introduced into prokaryotic and/or eukaryotic cells and, where appropriate, to be integrated into a genome. Vectors of this kind are preferably replicated and/or expressed in the cells. An intermediary vehicle may be adapted, for example, to the use in electroporation, in bombardment with microprojectiles, in liposomal administration, in the transfer with the aid of agrobacteria or in insertion via DNA or RNA viruses. Vectors comprise plasmids, phagemids, bacteriophages or viral genomes.

As used herein, the term "cell" preferably refers to a eukaryotic cell, more preferably a mammalian cell, most preferably a human cell.

According to the invention, the term "host cell" relates to any cell which can be transformed or transfected with an exogenous nucleic acid. The term "host cells" comprises according to the invention prokaryotic (e.g. *E. coli)* or eukaryotic cells (e.g. dendritic cells, B cells, CHO cells, COS cells, K562 cells, yeast cells and insect cells). Particular preference is given to mammalian cells such as cells from humans, mice, hamsters, pigs, goats, primates. The cells may be derived from a multiplicity of tissue types and comprise primary cells and cell lines. Specific examples comprise keratinocytes, peripheral blood leukocytes, stem cells of the bone marrow and embryonic stem cells. A nucleic acid may be present in the host cell in the form of a single copy or of two or more copies and, in one embodiment, is expressed in the host cell. Preferably, the host cell is not part of a human body.

According to the invention, the term "expression" is used in its most general meaning and comprises the production of RNA or of RNA and protein. It also comprises partial expression of nucleic acids. Furthermore, expression may be carried out transiently or stably. Preferred expression systems in mammalian cells comprise pcDNA3.1 and pRc/CMV (Invitrogen, Carlsbad, CA), which contain a selectable marker such as a gene imparting resistance to G418 (and thus enabling stably transfected cell lines to be selected) and the enhancer-promoter sequences of cytomegalovirus (CMV).

"Antisense nucleic acids" may be used for regulating, in particular reducing, expression of a nucleic acid. The term "antisense nucleic acid" refers according to the invention to an oligonucleotide which is an oligoribonucleotide, oligodeoxyribonucleotide, modified oligoribonucleotide or modified oligodeoxyribonucleotide and which hybridizes under physiological conditions to DNA comprising a particular gene or to mRNA of said gene, thereby inhibiting transcription of said gene and/or translation of said mRNA. An antisense nucleic acid may form a duplex with naturally occurring mRNA and thus prevent accumulation of or translation of the mRNA. Another possibility is the use of ribozymes for inactivating a nucleic acid.

Antisense oligonucleotides preferred according to the invention have a sequence of 6-50, in particular 10-30, 15-30 and 20-30, contiguous nucleotides of the target nucleic acid and preferably are fully complementary to the target nucleic acid or to a part thereof.

In preferred embodiments, the antisense oligonucleotide hybridizes with an N-terminal or 5' upstream site such as a translation initiation site, transcription initiation site or promoter site. In further embodiments, the antisense oligonucleotide hybridizes with a 3'-untranslated region or mRNA splicing site.

In one embodiment, an oligonucleotide such as an siRNA of the invention or an antisense nucleic acid of the invention consists of ribonucleotides, deoxyribonucleotides or a combination thereof, with the 5' end of one nucleotide and the 3' end of another nucleotide being linked to one another by a phosphodiester bond. These oligonucleotides may be synthesized in the conventional manner or produced recombinantly.

In preferred embodiments, the oligonucleotide is a "modified" oligonucleotide. Here, the oligonucleotide may be modified in very different ways, without impairing its ability to bind its target, in order to increase, for example, its stability or therapeutic efficacy. According to the invention, the term "modified oligonucleotide" means an oligonucleotide in which (i) at least two of its nucleotides are linked to one another by a synthetic internucleoside bond (i.e. an internucleoside bond which is not a phosphodiester bond) and/or (ii) a chemical group which is usually not found in nucleic acids is covalently linked to the oligonucleotide. Preferred synthetic internucleoside bonds are phosphorothioates, alkyl phosphonates, phosphorodithioates, phosphate esters, alkyl phosphonothioates, phosphoramidates, carbamates, carbonates, phosphate triesters, acetamidates, carboxymethyl esters and peptides.

The term "modified oligonucleotide" also comprises oligonucleotides having a covalently modified base and/or sugar. "Modified oligonucleotides" comprise, for example, oligonucleotides with sugar residues which are covalently bound to low molecular weight organic groups other than a hydroxyl group at the 3' position and a phosphate group at the 5' position. Modified oligonucleotides may comprise, for example, a 2'-O-alkylated ribose residue or another sugar instead of ribose, such as arabinose.

It is to be understood that all embodiments described above with respect to oligonucleotides may also apply to polynucleotides.

By "small interfering RNA" or "siRNA" as used herein is meant a molecule or complex comprising a sense RNA strand and a complementary antisense RNA strand annealed together by standard Watson-Crick base-pairing interactions (hereinafter "base-paired"). The sense strand and antisense strand of the siRNA preferably are greater than 10 nucleotides in length, more preferably greater than 15 nucleotides in length, and most preferably 18, 19, 20, 21, 22, 23, 24, 25, 26, 27, 28, 29, or 30 nucleotides in length. A range of 19-25 nucleotides is the most preferred size for siRNAs.

According to the invention, an siRNA specifically targets and causes RNAi-induced degradation of mRNA from a target gene, i.e. a target mRNA, so that the protein product of the target gene is not produced or is produced in reduced amounts.

siRNA according to the invention can comprise partially purified RNA, substantially pure RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end(s) of the siRNA or to one or more internal nucleotides of the siRNA; modifications that make the siRNA resistant to nuclease digestion (e.g., the use of 2'-substituted ribonucleotides or modifications to the sugar-phosphate backbone); or the substitution of one or more nucleotides in the siRNA with deoxyribonucleotides. Furthermore, siRNA may be modified to increase the stability thereof as described above for modified oligonucleotides, in particular by introducing one or more phosphorothioate linkages.

One or both strands of the siRNA can also comprise a 3'-overhang. As used herein, a "3'-overhang" refers to at least one unpaired nucleotide extending from the 3'-end of an RNA strand. Thus in one embodiment, the siRNA comprises at least one 3'-overhang of from 1 to about 6 nucleotides (which includes ribonucleotides or deoxynucleotides) in length, preferably from 1 to about 5 nucleotides in length, more preferably from 1 to about 4 nucleotides in length, and particularly preferably from about 2 to about 4 nucleotides in length. In the embodiment in which both strands of the siRNA molecule comprise a 3'-overhang, the length of the overhangs can be the same or different for each strand. In a most preferred embodiment, the 3'-overhang is present on both strands of the siRNA, and is 2 nucleotides in length. For example, each strand of the siRNA of the invention can comprise 3'-overhangs of dideoxythymidylic acid ("TT") or diuridylic acid ("uu").

In order to enhance the stability of the siRNA, the 3'-overhangs can also be stabilized against degradation. In one embodiment, the overhangs are stabilized by including purine nucleotides, such as adenosine or guanosine nucleotides. Alternatively, substitution of pyrimidine nucleotides by modified analogues, e.g., substitution of uridine nucleotides in the 3'-overhangs with 2'-deoxythymidine, is tolerated and does not affect the efficiency of RNAi degradation. In particular, the absence of a 2'-hydroxyl in the 2'-deoxythymidine significantly enhances the nuclease resistance of the 3'-overhang in tissue culture medium.

The sense and antisense strands of the siRNA can comprise two complementary, single-stranded RNA molecules or can comprise a single molecule in which two complementary portions are base-paired and are covalently linked by a single-stranded "hairpin" area. That is, the sense region and antisense region can be covalently connected via a linker molecule. The linker molecule can be a polynucleotide or non-nucleotide linker. Without wishing to be bound by any theory, it is believed that the hairpin area of the latter type of siRNA molecule is cleaved intracellularly by the "Dicer" protein (or its equivalent) to form an siRNA of two individual base-paired RNA molecules.

As used herein, "target mRNA" refers to an RNA molecule that is a target for downregulation. One skilled in the art would understand that the cDNA sequence is equivalent to the mRNA sequence, and can be used for the same purpose herein, i.e., the generation of siRNA.

siRNA can be expressed from pol III expression vectors without a change in targeting site, as expression of RNAs from pol III promoters is only believed to be efficient when the first transcribed nucleotide is a purine.

siRNA according to the invention can be targeted to any stretch of approximately 19-25 contiguous nucleotides in any of the target mRNA sequences (the "target sequence"). Techniques for selecting target sequences for siRNA are given, for example, in Tuschl T. et al., "The siRNA User Guide", revised Oct. 11, 2002, the entire disclosure of which is herein incorporated by reference. "The siRNA User Guide" is available on the world wide web at a website maintained by Dr. Thomas Tuschl, Laboratory of RNA Molecular Biology, Rockefeller University, New York, USA, and can be found by accessing the website of the Rockefeller University and searching with the keyword "siRNA". Thus, the sense strand of the present siRNA comprises a nucleotide sequence substantially identical to any contiguous stretch of about 19 to about 25 nucleotides in the target mRNA.

Generally, a target sequence on the target mRNA can be selected from a given cDNA sequence corresponding to the target mRNA, preferably beginning 50 to 100 nt downstream (i.e., in the 3'-direction) from the start codon. The target sequence can, however, be located in the 5'- or 3'-untranslated regions, or in the region nearby the start codon. For example, a suitable target sequence in the MS4A12 cDNA sequence is selected from the following group of target sequences:
(i) GATCATGGTTGGATTGATGCA (SEQ ID NO: 3)
(ii) GTCAACCGGGTCAAGGAAATA (SEQ ID NO: 6)

A preferred siRNA targeting the sequence (i), and which has 3'-overhangs on each strand (overhangs shown in bold), is:
5' ucaugguuggauugaugcaTT 3' (SEQ ID NO: 4)
3' CTaguaccaaccuaacuacgu 5' (SEQ ID NO: 5)

A preferred siRNA targeting the sequence (ii), and which has 3'-overhangs on each strand (overhangs shown in bold), is:
5' caaccgggucaaggaaauaTA 3' (SEQ ID NO: 7)
3' CAguuggcccaguuccuuuau 5' (SEQ ID NO: 8)

In the above list, all deoxyribonucleotides in a nucleic acid sequence are represented by capital letters (e.g., deoxythymidine is "T"), and ribonucleotides in a nucleic acid sequence are represented by lower case letters (e.g., uridine is "u").

It is understood that the target sequences given herein are with reference to the human MS4A12 cDNA, and thus these sequences contain deoxythymidines represented by "T". One skilled in the art would understand that, in the actual target sequence of the MS4A12 mRNA, the deoxythymidines would be replaced by uridines ("u"). Likewise, a target sequence contained within an siRNA of the invention would also contain uridines in place of deoxythymidines. siRNA can be obtained using a number of techniques known to those of skill in the art. For example, siRNA can be chemically synthesized or recombinantly produced using methods known in the art, such as the Drosophila in *vitro* system described in U.S. published application 2002/0086356 of Tuschl et al., the entire disclosure of which is herein incorporated by reference.

Preferably, siRNA is chemically synthesized using appropriately protected ribonucleoside phosphoramidites and a conventional DNA/RNA synthesizer. siRNA can be synthesized as two separate, complementary RNA molecules, or as a single RNA molecule with two complementary regions.

Alternatively, siRNA can also be expressed from recombinant circular or linear DNA plasmids using any suitable promoter. Such embodiments are included according to the present invention when reference is made herein to the administration of siRNA or the incorporation of siRNA into pharmaceutical compositions. Suitable promoters for expressing siRNA of the invention from a plasmid include, for example, the U6 or H1 RNA pol III promoter sequences and the cytomegalovirus promoter.

Selection of other suitable promoters is within the skill in the art. Nucleic acid molecules comprising a nucleic acid sequence for expressing an siRNA such as recombinant plasmids or viral vectors can also comprise inducible or regulatable promoters for expression of the siRNA in a particular tissue or in a particular intracellular environment.

The siRNA expressed from recombinant plasmids can either be isolated from cultured cell expression systems by standard techniques, or can be expressed intracellularly. siRNA can be expressed from a recombinant plasmid either as two separate, complementary RNA molecules, or as a single RNA molecule with two complementary regions.

Selection of plasmids suitable for expressing siRNA, methods for inserting nucleic acid sequences for expressing the siRNA into the plasmid, and methods of delivering the recombinant plasmid to the cells of interest are within the skill in the art. siRNA can also be expressed from recombinant viral vectors intracellularly in vivo. The recombinant viral vectors comprise sequences encoding the siRNA and any suitable promoter for expressing the siRNA sequences. The recombinant viral vectors can also comprise inducible or regulatable promoters for expression of the siRNA in a particular tissue or in a particular intracellular environment. siRNA can be expressed from a recombinant viral vector either as two separate, complementary RNA molecules, or as a single RNA molecule with two complementary regions.

The mRNA transcribed from the human MS4A12 gene can be analyzed for alternative splice forms using techniques well-known in the art. Such techniques include reverse transcription-polymerase chain reaction (RT-PCR), northern blotting and in-situ hybridization.

A technique called "RNAse protection" can also be used to identify alternatively spliced MS4A12 mRNAs. RNAse protection involves transcription of a gene sequence into synthetic RNA, which is hybridized to RNA derived from other cells; for example, cells which are induced to express MS4A12. The hybridized RNA is then incubated with enzymes that recognize RNA:RNA hybrid mismatches. Smaller than expected fragments indicate the presence of alternatively spliced mRNAs. The putative alternatively spliced mRNAs can be cloned and sequenced by methods well known to those skilled in the art.

RT-PCR can also be used to identify alternatively spliced MS4A12 mRNAs. In RT-PCR, mRNA from cells known to express MS4A12 is converted into cDNA by the enzyme reverse transcriptase, using methods well-known to those of ordinary skill in the art. The entire coding sequence of the cDNA is then amplified via PCR using a forward primer located in the 3'-untranslated region, and a reverse primer located in the 5'-untranslated region. The amplified products can be analyzed for alternative splice forms, for example by comparing the size of the amplified products with the size of the expected product from normally spliced mRNA, e.g., by agarose gel electrophoresis. Any change in the size of the amplified product can indicate alternative splicing.

The mRNA produced from mutant MS4A12 genes can also be readily identified with the techniques described above for identifying alternative splice forms. As used herein, "mutant" MS4A12 genes or mRNA include human MS4A12 genes or mRNA which differ in sequence from the MS4A12 sequences set forth herein. Thus, allelic forms of the MS4A12 gene, and the mRNA produced from them, are considered "mutants" for purposes of this invention. "Reduce" or "inhibit" as used herein means the ability to cause an overall decrease, preferably of 20% or greater, more preferably of 50% or greater, and most preferably of 75%, 90% or 95% or greater, in the level, e.g. in the level of protein or mRNA as compared to a reference sample (e.g., a sample not treated with siRNA). This reduction or inhibition of RNA or protein expression can occur through targeted mRNA cleavage or degradation. Assays for protein expression or nucleic acid expression are known in the art and include, for example, ELISA, western blot analysis for protein expression, and northern blotting or RNase protection assays for RNA. In certain embodiments, the term "inhibit" or "inhibition" refers to a complete inhibition, i.e. a decrease of 100% in the level.

The term "peptide" comprises oligo- and polypeptides and refers to substances comprising two or more, preferably 3 or more, preferably 4 or more, preferably 6 or more, preferably 8 or more, preferably 10 or more, preferably 13 or more, preferably 16 more, preferably 21 or more and up to preferably 8, 10, 20, 30, 40 or 50, in particular 100 amino acids joined covalently by peptide bonds. The term "protein" refers to large peptides, preferably to peptides with more than 100 amino acid residues, but in general the terms "peptides" and "proteins" are synonyms and are used interchangeably herein.

Preferably, the proteins and peptides described according to the invention have been isolated. The terms "isolated protein" or "isolated peptide" mean that the protein or peptide has been separated from its natural environment. An isolated protein or peptide may be in an essentially purified state. The term "essentially purified" means that the protein or peptide is essentially free of other substances with which it is associated in nature or in vivo.

Such proteins and peptides may be used, for example, in producing antibodies and in an immunological or diagnostic assay or as therapeutics. Proteins and peptides described according to the invention may be isolated from biological samples such as tissue or cell homogenates and may also be expressed recombinantly in a multiplicity of pro- or eukaryotic expression systems.

For the purposes of the present invention, "derivatives" of a protein or peptide or of an amino acid sequence comprise amino acid insertion variants, amino acid deletion variants and/or amino acid substitution variants.

Amino acid insertion variants comprise amino- and/or carboxy-terminal fusions and also insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible.

Amino acid deletion variants are characterized by the removal of one or more amino acids from the sequence.

Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties.

Preferably, a "derivative" of a protein or peptide or of an amino acid sequence relates to a variant wherein up to 50, more preferably up to 20, up to 15, up to 10, up to 8, up to 5, up to 4, up to 3, up to 2 or only 1 amino acid(s) are/is inserted, deleted and/or substituted.

"Conservative substitutions" may be made, for instance, on the basis of similarity in polarity, charge, solubility, hydrophobicity, hydrophilicity, and/or the amphipathic nature of the residues involved. For example: (a) nonpolar (hydrophobic) amino acids include alanine, leucine, isoleucine, valine, proline, phenylalanine, tryptophan, and methionine; (b) polar neutral amino acids include glycine, serine, threonine, cysteine, tyrosine, asparagine, and glutamine; (c) positively charged (basic) amino acids include arginine, lysine, and histidine; and (d) negatively charged (acidic) amino acids include aspartic acid and glutamic acid. Substitutions typically may be made within groups (a)-(d). In addition, glycine and proline may be substituted for one another based on their ability to disrupt α-helices. Some preferred substitutions may be made among the following groups: (i) S and T; (ii) P and G; and (iii) A, V, L and I. Given the known genetic code, and recombinant and synthetic DNA techniques, the skilled scientist readily can construct DNAs encoding the conservative amino acid variants.

Preferably, the degree of similarity, preferably identity between a specific amino acid sequence described herein and an amino acid sequence which is a derivative of said specific amino acid sequence will be at least 70%, preferably at least 80%, preferably at least 85%, even more preferably at least 90% or most preferably at least 95%, 96%, 97%, 98% or 99%. The degree of similarity or identity is given preferably for a region of at least about 20, at least about 40, at least about 60, at least about 80, at least about 100, at least about 120, at least about 140, at least about 160, at least about 200 or 250 amino acids. In preferred embodiments, the degree of similarity or identity is given for the entire length of the reference amino acid sequence.

The amino acid variants described above may be readily prepared with the aid of known peptide synthesis techniques such as, for example, by solid phase synthesis (Merrifield, 1964) and similar methods or by recombinant DNA manipulation. The manipulation of DNA sequences for preparing proteins and peptides having substitutions, insertions or deletions, is described in detail in Sambrook et al. (1989), for example.

According to the invention, "derivatives" of proteins and peptides also comprise single or multiple substitutions, deletions and/or additions of any molecules associated with the protein or peptide, such as carbohydrates, lipids and/or proteins or peptides. The term "derivative" also extends to all functional chemical equivalents of said proteins and peptides.

According to the invention, a part or fragment of a protein or peptide preferably has a functional property of the protein or peptide from which it has been derived. Such functional properties comprise the interaction with antibodies, the interaction with other peptides or proteins, the selective binding of nucleic acids and an enzymatic activity. A part or fragment of a protein or peptide preferably comprises a sequence of at least 6, in particular at least 8, at least 10, at least 12, at least 15, at least 20, at least 30 or at least 50, consecutive amino acids of the protein or peptide. A part or fragment of a protein or peptide preferably comprises a sequence of up to 8, in particular up to 10, up to 12, up to 15, up to 20, up to 30 or up to 55, consecutive amino acids of the protein or peptide. A part or fragment of a protein or peptide is preferably a part of the protein or peptide which corresponds to the non-transmembrane portion, in particular the extracellular portion of the protein or peptide, or is comprised thereof.

A part or a fragment of a nucleic acid coding for a protein or peptide relates according to the invention to the part of the nucleic acid, which codes at least for the protein or peptide and/or for a part or a fragment of said protein or peptide as defined above. A part or fragment of a nucleic acid coding for a protein or peptide is preferably that part of the nucleic acid corresponding to the open reading frame.

According to the invention, the term "binding" preferably relates to a specific binding. "Specific binding" means that an agent such as an antibody binds stronger to a target such as an epitope for which it is specific compared to the binding to another target. An agent binds stronger to a first target compared to a second target if it binds to the first target with a dissociation constant (K_{D}) which is lower than the dissociation constant for the second target. Preferably the dissociation constant (K_{D}) for the target to which the agent binds specifically is more than 10-fold, preferably more than 20-fold, more preferably more than 50-fold, even more preferably more than 100-fold, 200-fold, 500-fold or 1000-fold lower than the dissociation constant (K_{D}) for the target to which the agent does not bind specifically.

According to the invention, a specific antibody relates to an antibody which specifically binds to its target. Such specific antibodies may, for example, be obtained by immunization with its target, for example MS4A12 protein or a part thereof such as the extracellular regions thereof.

Antisera which contain specific antibodies specifically binding to the target protein can be prepared by various standard processes; see, for example, "Monoclonal Antibodies: A Practical Approach" by Philip Shepherd, Christopher Dean ISBN 0-19-963722-9; "Antibodies: A Laboratory Manual" by Ed Harlow, David Lane, ISBN: 0879693142 and "Using Antibodies: A Laboratory Manual: Portable Protocol NO" by Edward Harlow, David Lane, Ed Harlow ISBN 0879695447. Thereby it is also possible to generate affine and specific antibodies which recognize complex membrane proteins in their native form (Azorsa et al., J. Immunol. Methods 229: 35-48, 1999; Anderson et al., J. Immunol. 143: 1899-1904, 1989; Gardsvoll, J. Immunol. Methods 234: 107-116, 2000). This is in particular relevant for the preparation of antibodies which are to be used therapeutically. In this respect, it is possible to immunize with the whole protein, with extracellular partial sequences as well as with cells which express the target molecule in physiologically folded form.

Monoclonal antibodies are traditionally prepared using the hybridoma technology (for technical details see: "Monoclonal Antibodies: A Practical Approach" by Philip Shepherd, Christopher Dean ISBN 0-19-963722-9; "Antibodies: A Laboratory Manual" by Ed Harlow, David Lane ISBN: 0879693142; "Using Antibodies: A Laboratory Manual: Portable Protocol NO" by Edward Harlow, David Lane, Ed Harlow ISBN: 0879695447).

It is known that only a small part of an antibody molecule, the paratope, is involved in binding of the antibody to its epitope (cf. Clark, W.R. (1986), The Experimental Foundations of Modern Immunology, Wiley & Sons, Inc., New York; Roitt, I. (1991), Essential Immunology, 7th Edition, Blackwell Scientific Publications, Oxford). The pFc' and Fc regions are, for example, effectors of the complement cascade but are not involved in antigen binding. An antibody from which the pFc' region has been enzymatically removed or which has been produced without the pFc' region, referred to as F(ab')₂ fragment, carries both antigen binding sites of a complete antibody. Similarly, an antibody from which the Fc region has been enzymatically removed or which has been produced without said Fc region, referred to as Fab fragment, carries one antigen binding site of an intact antibody molecule. Furthermore, Fab fragments consist of a covalently bound light chain of an antibody and part of the heavy chain of said antibody, referred to as Fd. The Fd fragments are the main determinants of antibody specificity (a single Fd fragment can be associated with up to ten different light chains, without altering the specificity of the antibody) and Fd fragments, when isolated, retain the ability to bind to an epitope.

Located within the antigen-binding part of an antibody are complementary-determining regions (CDRs) which interact directly with the antigen epitope and framework regions (FRs) which maintain the tertiary structure of the paratope. Both the Fd fragment of the heavy chain and the light chain of IgG immunoglobulins contain four framework regions (FR1 to FR4) which are separated in each case by three complementary-determining regions (CDR1 to CDR3). The CDRs and, in particular, the CDR3 regions and, still more particularly, the CDR3 region of the heavy chain are responsible to a large extent for antibody specificity.

Non-CDR regions of a mammalian antibody are known to be able to be replaced by similar regions of antibodies with the same or a different specificity, with the specificity for the epitope of the original antibody being retained. This made possible the development of "humanized" antibodies in which nonhuman CDRs are covalently linked to human FR and/or Fc/pFc' regions to produce a functional antibody.

As another example, WO 92/04381 describes the production and use of humanized murine RSV antibodies in which at least part of the murine FR regions have been replaced with FR regions of a human origin. Antibodies of this kind, including fragments of intact antibodies with antigen-binding capability, are often referred to as "chimeric" antibodies.

According to the invention, the term "antibody" also includes F(ab')₂, Fab, Fv, and Fd fragments of antibodies, chimeric antibodies, in which the Fc and/or FR and/or CDR1 and/or CDR2 and/or light chain-CDR3 regions have been replaced with homologous human or nonhuman sequences, chimeric F(ab')₂-fragment antibodies in which the FR and/or CDR1 and/or CDR2 and/or light chain-CDR3 regions have been replaced with homologous human or nonhuman sequences, chimeric Fab-fragment antibodies in which the FR and/or CDR1 and/or CDR2 and/or light chain-CDR3 regions have been replaced with homologous human or nonhuman sequences, and chimeric Fd-fragment antibodies in which the FR and/or CDR1 and/or CDR2 regions have been replaced with homologous human or nonhuman sequences. The term "antibody" also comprises "single-chain" antibodies.

Antibodies may also be coupled to specific marker substances for displaying cells and tissues expressing the target antigen. They may also be coupled to therapeutically useful substances.

Marker substances include any label that functions to: (i) provide a detectable signal; (ii) interact with a second label to modify the detectable signal provided by the first or second label, e.g. FRET (Fluorescence Resonance Energy Transfer); (iii) affect mobility, e.g. electrophoretic mobility, by charge, hydrophobicity, shape, or other physical parameters, or (iv) provide a capture moiety, e.g., affinity, antibody/antigen, or ionic complexation. Suitable as label are structures, such as fluorescent labels, luminescent labels, chromophore labels, radioisotopic labels, isotopic labels, preferably stable isotopic labels, isobaric labels, enzyme labels, particle labels, in particular metal particle labels, magnetic particle labels, polymer particle labels, small organic molecules such as biotin, ligands of receptors or binding molecules such as cell adhesion proteins or lectins, label-sequences comprising nucleic acids and/or amino acid residues which can be detected by use of binding agents, etc. Marker substances comprise, in a nonlimiting manner, barium sulfate, iocetamic acid, iopanoic acid, calcium ipodate, sodium diatrizoate, meglumine diatrizoate, metrizamide, sodium tyropanoate and radio diagnostic, including positron emitters such as fluorine-18 and carbon-11, gamma emitters such as iodine-123, technetium-99m, iodine-131 and indium-111, nuclides for nuclear magnetic resonance, such as fluorine and gadolinium.

According to the invention, a "small molecule compound" is a compound having a low molecular mass, preferably a molecular mass of up to 20.000 Da, more preferably up to 15.000 Da, up to 10.000 Da, up to 7.500 Da, up to 5.000 Da, up to 2.000 Da or up to 1.000 Da. Preferably, the small molecule compound is an organic compound. A small molecule compound may be a modified or unmodified naturally occurring compound or a chemically synthesized compound. Preferably, the small molecule compound is not a polymeric compound. However, the small molecule compound may also be a polynucleotide or a polypeptide.

According to the invention, an EGF receptor is a receptor for binding of epidermal growth factor (EGF). Preferably, an EGF receptor is located at the plasma membrane, and is more preferably a transmembrane protein. Preferably, an EGF receptor is a tyrosine kinase receptor and the activity is phosphorylation of target proteins. Preferred EGF receptors are human EGFR, human ErbB-1, or human HER1. However, the EGF receptor may also be a species homolog of these human EGF receptors.

According to the invention, the term "therapeutically useful substance" means any molecule which may exert a therapeutic effect. According to the invention, a therapeutically useful substance includes anticancer agents, radioactive iodine-labeled compounds, toxins, cytostatic or cytolytic drugs, etc. Anticancer agents comprise, for example, aminoglutethimide, azathioprine, bleomycin sulfate, busulfan, carmustine, chlorambucil, cisplatin, cyclophosphamide, cyclosporine, cytarabidine, dacarbazine, dactinomycin, daunorubin, doxorubicin, taxol, etoposide, fluorouracil, interferon-α, lomustine, mercaptopurine, methotrexate, mitotane, procarbazine HCl, thioguanine, vinblastine sulfate and vincristine sulfate. Other anticancer agents are described, for example, in Goodman and Gilman, "The Pharmacological Basis of Therapeutics", 8th Edition, 1990, McGraw-Hill, Inc., in particular Chapter 52 (Antineoplastic Agents (Paul Calabresi and Bruce A. Chabner). Toxins may be proteins such as pokeweed antiviral protein, cholera toxin, pertussis toxin, ricin, gelonin, abrin, diphtheria exotoxin or *Pseudomonas* exotoxin. Toxin residues may also be high energy-emitting radionuclides such as cobalt-60.

The term "patient" means according to the invention a human being, a nonhuman primate or another animal, in particular a mammal such as a cow, horse, pig, sheep, goat, dog, cat or a rodent such as a mouse and rat. In a particularly preferred embodiment, the patient is a human being.

According to the invention the term "increased" or "increased amount" preferably refers to an increase by at least 10%, in particular at least 20%, at least 50% or at least 100%. Furthermore, the term "increase" may also refer to a situation where an effect or amount which is to be increased was not present, i.e. zero, prior to the increase, but is present, i.e. greater than zero, following the increase.

According to the invention, the term "cancer" comprises any cancer and preferably refers to cancer characterized by the expression of MS4A12, i.e., at least a part of the cancer cells of the cancer express MS4A12. Furthermore, the cancer preferably is characterized by the expression of an EGF receptor, i.e., at least a part of the cancer cells express an EGF receptor. In a more preferred embodiment, the cancer is characterized by the expression of an EGF receptor and the expression of an EGF receptor, i.e., at least a part of the cancer cells of the cancer express MS4A12 and at least a part of the cancer cells express an EGF receptor. Most preferably, at least a part of the cancer cells express MS4A12 and an EGF receptor.

According to the invention, the term "cancer" comprises metastases of the cancer. In preferred embodiments, the cancer is colon cancer and cancer metastases are colon cancer metastases, i.e. cancer metastases derived from a colon cancer. Preferably, a cancer is a carcinoma such as a colon carcinoma, in particular an adenocarcinoma such as an adenocarcinoma of the colon.

The terms "colon cancer" and "colorectal cancer" are used interchangeably herein and include cancers of the colon as well as cancers of the rectum. Similarly, the terms "colon tumor" and "colon carcinoma" also include "colorectal tumor" and "colorectal carcinoma", respectively.

By "tumor" is meant an abnormal group of cells or tissue that grows by a rapid, uncontrolled cellular proliferation and continues to grow after the stimuli that initiated the new growth cease. Tumors show partial or complete lack of structural organization and functional coordination with the normal tissue, and usually form a distinct mass of tissue, which may be either benign or malignant. Preferably, according to the invention a tumor is a colon tumor.

According to the invention, "neoplastic cells" are cells showing abnormally increased cellular proliferation. According to the invention, a "cancer cell" is a cell involved in a cancer disease and includes tumor cells, leukemia cells and cells of a metastasis. According to the invention, a "tumor cell" is a cell present in a tumor tissue. Preferably, a "cancer cell" or "tumor cell" is a neoplastic cell. The term "at least a part of the cancer cells" preferably refers to at least 5%, at least 10%, at least 20%, at least 30%, at least 40%, at least 50%, at least 60%, at least 70%, at least 80%, at least 90%, at least 95% or about 100% of the cancer cells.

By "metastasis" is meant the spread of cancer cells from its original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor at the target site depends on angiogenesis. Tumor metastasis often occurs even after the removal of the primary tumor because tumor cells or components may remain and develop metastatic potential. In one embodiment, the term "metastasis" according to the invention relates to "distant metastasis" which relates to a metastasis which is remote from the primary tumor and the regional lymph node system.

According to the invention, a "tumor sample" is a sample which contains one or more tumor cells. A tumor sample may be a tissue sample, including bodily fluids, and/or a cellular sample and may be obtained in the conventional manner such as by tissue biopsy, including punch biopsy, and by taking blood, bronchial aspirate, sputum, urine, feces or other body fluids. According to the invention, the term "tumor sample" also includes fractions of tumor samples. Preferably, the tumor sample is obtained from a patient having a tumor, and preferably is isolated from said patient.

The invention also provides for administration of nucleic acids, proteins or peptides. Nucleic acids, proteins and peptides may be administered in a manner known per se. For example, nucleic acids may be administered in vivo by using vectors such as viruses and target-controlled liposomes. If according to the invention reference is made to the administration or incorporation into pharmaceutical compositions of nucleic acids this includes embodiments wherein the nucleic acid is present in such vectors.

In a preferred embodiment, a virus or viral vector for administering a nucleic acid is selected from the group consisting of adenoviruses, adeno-associated viruses, pox viruses, including vaccinia virus and attenuated pox viruses, Semliki Forest virus, retroviruses, Sindbis virus and Ty virus-like particles. Particular preference is given to adenoviruses and retroviruses. The retroviruses are typically replication-deficient (i.e. they are incapable of generating infectious particles).

Methods of introducing nucleic acids into cells in vitro or in vivo comprise transfection of nucleic acid calcium phosphate precipitates, transfection of nucleic acids associated with DEAE, transfection or infection with the above viruses carrying the nucleic acids of interest, liposome-mediated transfection, and the like. In particular embodiments, preference is given to directing the nucleic acid to particular cells. In such embodiments, a carrier used for administering a nucleic acid to a cell (e.g. a retrovirus or a liposome) may have a bound target control molecule. For example, a molecule such as an antibody specific for a surface membrane protein on the target cell or a ligand for a receptor on the target cell may be incorporated into or attached to the nucleic acid carrier. If administration of a nucleic acid via liposomes is desired, proteins binding to a surface membrane protein associated with endocytosis may be incorporated into the liposome formulation in order to make target control and/or uptake possible. Such proteins comprise capsid proteins or fragments thereof which are specific for a particular cell type, antibodies to proteins which are internalized, proteins addressing an intracellular site, and the like.

The therapeutic compositions of the invention may be administered in pharmaceutically compatible preparations. Such preparations may usually contain pharmaceutically compatible concentrations of salts, buffer substances, preservatives, carriers, supplementing immunity-enhancing substances such as adjuvants, e.g. CpG oligonucleotides, cytokines, chemokines, saponin, GM-CSF and/or RNA and, where appropriate, other therapeutically active compounds.

The therapeutically active agents of the invention may be administered via any conventional route, including by injection or infusion. The administration may be carried out, for example, orally, intravenously, intraperitonealy, intramuscularly, subcutaneously, transdermally or intrarectal. Antisense nucleic acids are preferably administered by slow intravenous administration.

The compositions of the invention are administered in effective amounts. An "effective amount" refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition. According to the invention, a treatment of cancer may also include the treatment of cancer metastases which have already formed or will form. According to the invention, the term "treatment" comprises therapeutic and prophylactic treatment, i.e. prevention.

An effective amount of a composition of the invention will depend on the condition to be treated, the severeness of the disease, the individual parameters of the patient, including age, physiological condition, size and weight, the duration of treatment, the type of an accompanying therapy (if present), the specific route of administration and similar factors.

The pharmaceutical compositions of the invention are preferably sterile and contain an effective amount of the therapeutically active agent to generate the desired reaction or the desired effect.

The doses administered of the compositions of the invention may depend on various parameters such as the type of administration, the condition of the patient, the desired period of administration, etc. In the case that a reaction in a patient is insufficient with an initial dose, higher doses (or effectively higher doses achieved by a different, more localized route of administration) may be used.

Generally, doses of the agent of the invention of from 1 ng to 1 mg, preferably from 10 ng to 100 µg, are formulated and administered for a treatment or for generating or increasing an immune response. If the administration of nucleic acids (DNA and RNA) such as siRNA, antisense nucleic acids, or nucleic acid molecules coding for antibodies is desired, doses of from 1 ng to 0.1 mg are formulated and administered.

The pharmaceutical compositions of the invention are generally administered in pharmaceutically compatible amounts and in pharmaceutically compatible compositions. The term "pharmaceutically compatible" refers to a nontoxic material which does not interact with the action of the active component of the pharmaceutical composition. Preparations of this kind may usually contain salts, buffer substances, preservatives, carriers and, where appropriate, other therapeutically active compounds. When used in medicine, the salts should be pharmaceutically compatible. However, salts which are not pharmaceutically compatible may used for preparing pharmaceutically compatible salts and are included in the invention. Pharmacologically and pharmaceutically compatible salts of this kind comprise in a nonlimiting way those prepared from the following acids: hydrochloric, hydrobromic, sulfuric, nitric, phosphoric, maleic, acetic, salicylic, citric, formic, malonic, succinic acids, and the like. Pharmaceutically compatible salts may also be prepared as alkali metal salts or alkaline earth metal salts, such as sodium salts, potassium salts or calcium salts.

A pharmaceutical composition of the invention may comprise a pharmaceutically compatible carrier.

According to the invention, the term "pharmaceutically compatible carrier" refers to one or more compatible solid or liquid fillers, diluents or encapsulating substances, which are suitable for administration to humans. The term "carrier" refers to an organic or inorganic component, of a natural or synthetic nature, in which the active component is combined in order to facilitate application. The components of the pharmaceutical composition of the invention are usually such that no interaction occurs which substantially impairs the desired pharmaceutical efficacy.

The pharmaceutical compositions of the invention may contain suitable buffer substances such as acetic acid in a salt, citric acid in a salt, boric acid in a salt and phosphoric acid in a salt.

The pharmaceutical compositions may, where appropriate, also contain suitable preservatives such as benzalkonium chloride, chlorobutanol, paraben and thimerosal.

The pharmaceutical compositions are usually provided in a uniform dosage form and may be prepared in a manner known per se. Pharmaceutical compositions of the invention may be in the form of capsules, tablets, suppositories, lozenges, solutions, suspensions, syrups, elixirs or in the form of an emulsion, for example.

Compositions suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

The present invention is described in detail by the figures and examples below, which are used only for illustration purposes and are not meant to be limiting. Owing to the description and the examples, further embodiments which are likewise included in the invention are accessible to the skilled worker.

### Figures:

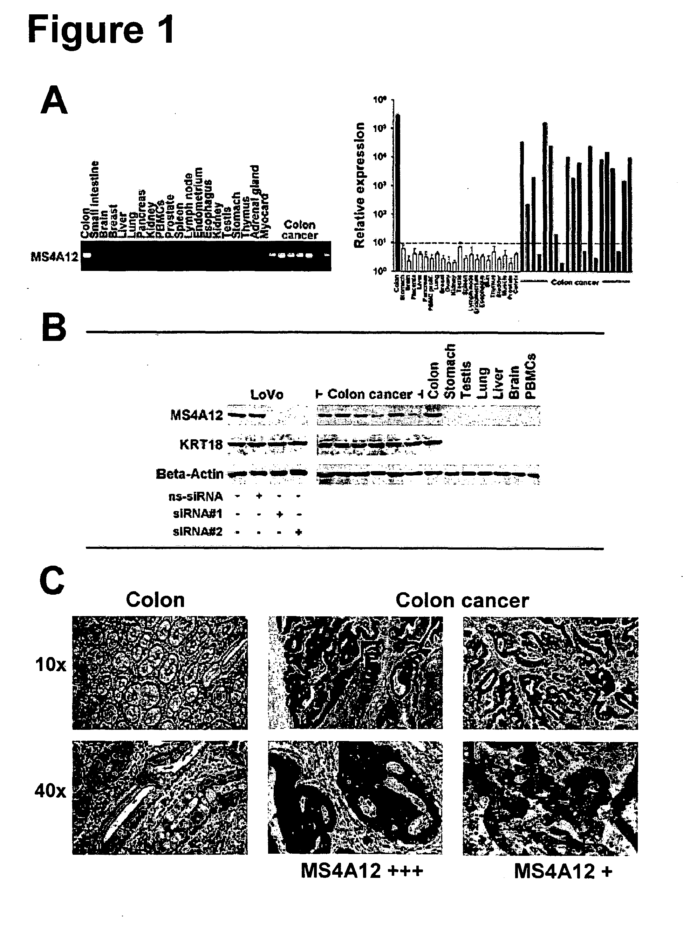

### Fig. 1. MS4A12 expression is restricted to normal colonic mucosa and colon cancer.

(A) Endpoint RT-PCR (left) and quantitative real-time RT-PCR (right) of MS4A12 in normal tissues and colon cancer. For each normal tissue the mean expression values of up to five individual tissue specimens are shown. Error bars, STD; dashed line, expression cut-off. (B) Silencing of MS4A12 protein expression in LoVo colon cancer cells 48 h after transfection with two individual siRNA duplexes. ns-siRNA, non-silencing siRNA (left). MS4A12 protein expression in normal tissues and colon cancer was detected with anti-MS4A12/N-term antiserum (right). Beta-Actin was used as loading control and Cytokeratin18 (KRT18) was used as a marker specific for epithelial cells. (C) IHC detection of MS4A12 protein expression in normal colon and colon cancers with high (MS4A12+++) and low (MS4A12+) expression.

### Fig. 2. MS4A12 is a CD20-like plasma membrane protein.

(A) Predicted plasma membrane topology of MS4A12. The region with high homology to CD20 is boxed gray. (B) IF microscopic analysis of CHO cells transfected with MS4A12-eGFP stained with anti-MS4A12/N-term antiserum.
(C) IF microscopic analysis of LoVo cells stained with anti-MS4A12/N-term 48h after transfection with control siRNA or MS4A12-specific siRNAs. (D) FACS analysis of CHO cells transfected with N- or C-terminally eGFP-tagged MS4A12. Non-permeabilized and permeabilized cells were stained with a rabbit polyclonal anti-eGFP antibody and an anti-rabbit Cy5-labeled secondary antibody.

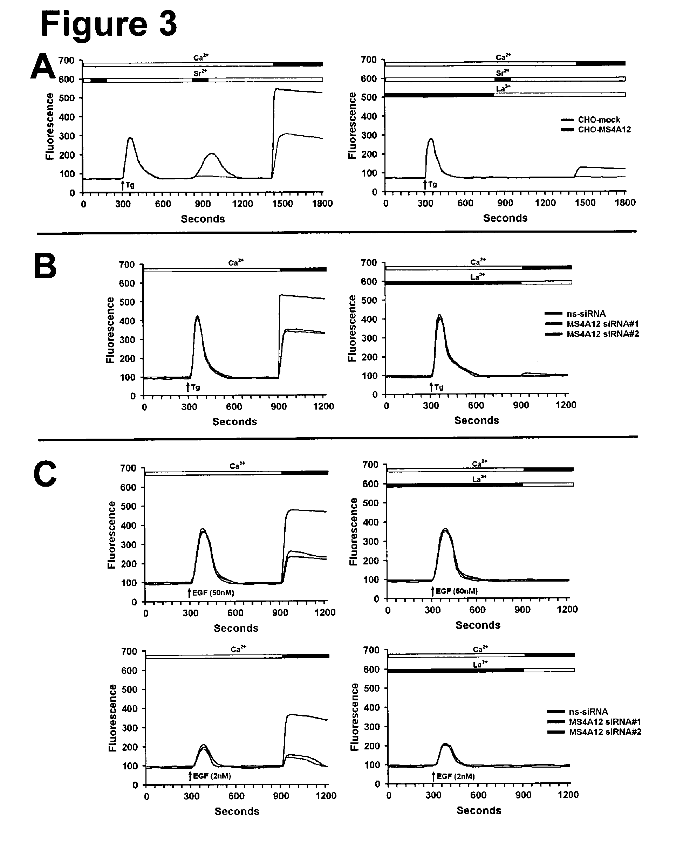

### Fig. 3. Store-operated calcium entry mediated by MS4A12.

(A) Ca²⁺ (1mM) and Sr²⁺ (1mM) entry in CHO cells transfected with MS4A12 or with vector alone as control was measured after Ca²⁺ store-depletion with Tg (300nM) without (left) and with (right) pretreatment of cells with known SOC blocker La³⁺ (25µM). (B) Ca²⁺ (1mM) entry in LoVo cells after Ca²⁺ store-depletion with Tg (300nM) without (left) and with (right) pretreatment of cells with La3⁺ (25µM). (C) Ca²⁺ entry measured after Ca²⁺ store-depletion in response to EGF (50nM, upper row; 2nM, lower row) without (left) and with (right) pretreatment of cells with La3⁺ (25µM). (Shown are representative results of three independent experiments done in triplicates.)

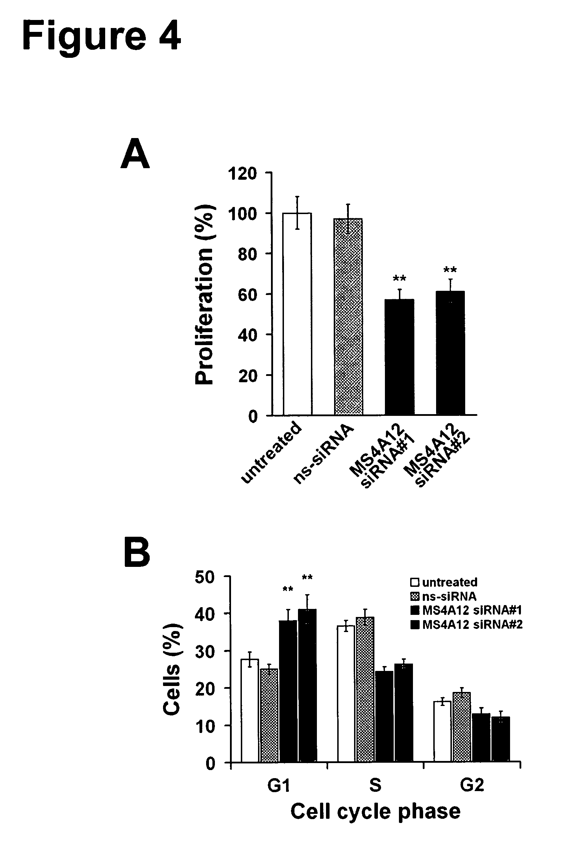

### Fig. 4. MS4A12 silencing impairs proliferation of LoVo colon cancer cells.

(A) 48h after transfection with siRNAs LoVo cells were cultured with 50 nM EGF for 48h. Proliferation was measured by incorporation of BrdU into newly synthesized DNA. (B) Cell cycle analysis of LoVo cells cultured under the same conditions shown as bar chart of cell fractions in different cell cycle states. All experiments were done in triplicates and repeated twice, shown are the mean values of all experiments +/-STD. * p<0.05, ** p<0.005.

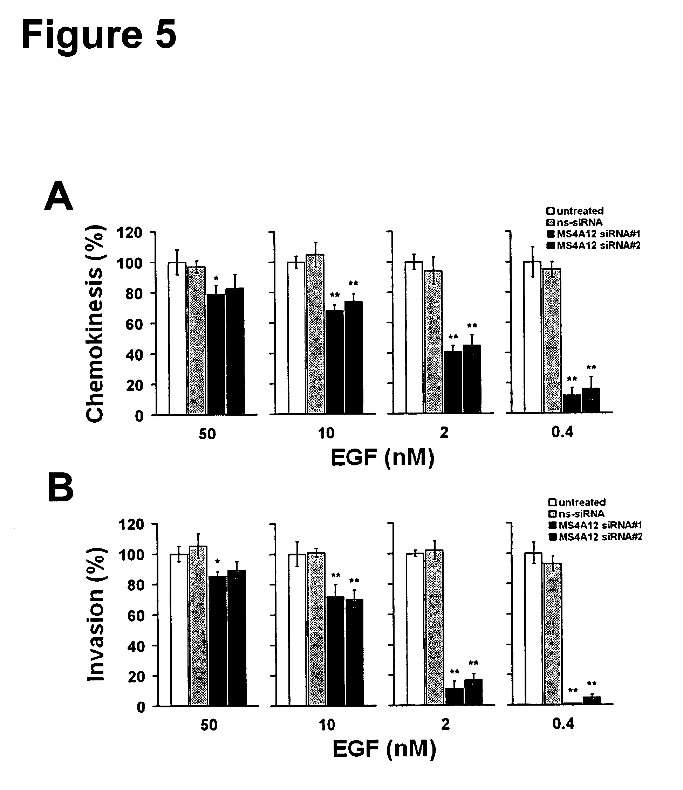

### Fig. 5. MS4A12 silencing impairs motility and invasion of LoVo colon cancer cells.

(A) Chemokinesis (cell motility) was analyzed in transwell migration assays with various concentrations of EGF added to the upper as well as the lower chamber was analyzed after 12 h. (B) Analysis of chemotactic invasion into Matrigel 24 h after various concentrations of EGF as chemoattractant have been added to the lower chamber. Data are normalized to the non-transfected control cells for each concentration of EGF to emphasize that MS4A12 knock down has the most prominent impact on motility and invasion at low EGF concentrations. All experiments were done in triplicates and repeated twice, shown are the mean values of all experiments +/- STD. * p<0.05, ** p<0.005.

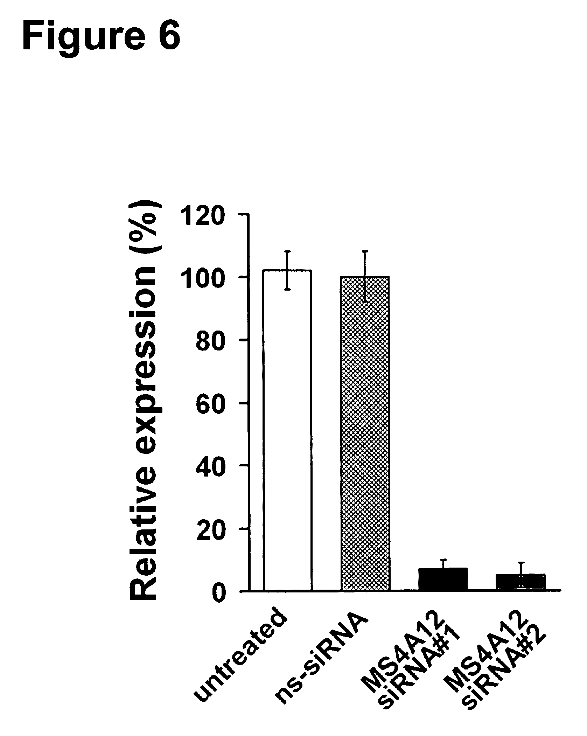

### Fig. 6. Quantification of MS4A12 transcript knock down using siRNA

MS4A12 transcript knock down in cells of the MS4A12 expressing colon cancer cell line LoVo was quantified by real-time RT-PCR 48h after transfection with two individual MS4A12 specific siRNA duplexes. Stable and reproducible reduction of constitutive MS4A12 mRNA expression by 90% was observed, whereas scrambled non-silencing control duplexes had no effect.

### Fig. 7. IHC analysis of MS4A12 expression in normal tissues.

Immunohistochemistry with anti-MS4A12/N-term antibody on sections obtained from testis and gastrointestinal tissues other than normal colon mucosa showed no detectable MS4A12 staining, excluding that smaller subpopulations present within these composite organ tissues do express this protein.

### Fig. 8. Amino acid sequence alignments of MS4A12

(A) Alignment of the MS4A12 protein sequence with other family members of the CD20 family. MS4A1: SEQ ID NO: 17; MS4A2: SEQ ID NO: 18; MS4A3: SEQ ID NO: 19; MS4A12: SEQ ID NO: 2. (B) Alignment of the MS4A12 protein sequence with the CD20 domain (pfam04103; SEQ ID NO: 20).

### Examples:

The techniques and methods mentioned herein are carried out in a manner known per se and are described, for example, in Sambrook et al., Molecular Cloning: A Laboratory Manual, 2nd Edition (1989) Cold Spring Harbor Laboratory Press, Cold Spring Harbor, N.Y. All methods including the use of kits and reagents are carried out according to the manufacturers' information unless specifically indicated.

### Screening for placenta-specific genes aberrantly activated in tumors

### Tissues and cell lines

Recombinant DNA work was done with the official permission and according to the rules of the state government of Rhineland-Palatinate. Fresh-frozen tissues were obtained as human surplus materials during routine diagnostic or therapeutic procedures. Precautions were taken to avoid prolonged exposure and tissues were stored at -80°C until use. Colon cancer cell line LoVo was cultured in DMEM + 10% FBS.

### Data mining strategy for discovery

As a first step, we created a general human expressed sequence tag (EST) file from all available libraries that contained the keyword "Homo sapiens" in the organism field of the dbEST report file (ftp://ncbi.nlm.nih.gov/repository/dbEST). To reduce complexity of the approach only libraries representing more than 100 ESTs and only ESTs encoding known full length genes were considered. Next, a colon tissue specific subfile was generated by extracting all those ESTs from the general file that contained the words "colon" in the library name, organism, tissue type, organ, or cell line field. In total, we analyzed 6,280,883 ESTs represented in 4,021 cDNA libraries. To account for the fact, that several EST may be derived from the same gene, the colon tissue ESTs were grouped into clusters assembling ESTs that shared one or more stretches of high sequence identity. The sequence homology searching program blastn (www.ncbi.nlm.nih.gov/BLAST) was run sequentially with the homology stringency set at S = 300; V = 300; B = 300; n = -20 for each sequence recorded in the colon tissue EST subfile against all of the human ESTs in the general list. For each query EST from the colon tissue subfile, the search extracted a list of EST entries (hits) from the general EST file, each of them annotated with its tissue origin, providing an "electronic Northern". Determination of the number of different non-colon tissues represented in such a hit list was used as a measure for the extent of colon tissue specificity of each individual EST. All candidates obtained by this procedure were subjected to motif and structure analysis by SMART (http://smart.embl.de/smart) to identify potential surface molecules. Candidates obtained by this procedure were subjected to motif and structure analysis by SMART to identify surface molecules topologically resembling CD20 (minimal requirements: at least four hydrophobic regions qualifying as transmembrane domains according to TMHMM, and prediction of at least one extracellular loop).

### RNA-Isolation, RT-PCR and real-time RT-PCR

RNA extraction, first-strand cDNA synthesis, RT-PCR and real-time RT-PCR were performed as previously described (Koslowski et al., Cancer Res. 64: 5988-93, 2004). Briefly, total cellular RNA was extracted from frozen tissue specimens using RNeasy Mini Kit (Qiagen), primed with a dT18 oligonucleotide and reverse transcribed with Superscript II (Invitrogen-Life Technologies, Inc.) according to the manufacturer's instructions. Integrity of the obtained cDNA was tested by amplification of p53 transcripts in a 30-cycle PCR (sense, 5'-CGT GAG CGC TTC GAG ATG TTC CG-3' (SEQ ID NO: 15); antisense, 5'-CCT AAC CAG CTG CCC AAC TGT AG-3' (SEQ ID NO: 16); annealing temperature 67°C).

For end-point analysis MS4A12-specific oligonucleotides (sense 5'-GAG CTT TCC CGT TGT CTG GTG-3' (SEQ ID NO: 11); antisense 5'-GCT GAA GAA GAC GCT GGT GTC-3' (SEQ ID NO: 12), 60°C annealing) were used in a 35 cycle RT-PCR. Real-time quantitative expression analysis was performed in triplicates in a 40 cycle RT-PCR. After normalization to HPRT (sense 5'-TGA CAC TGG CAA AAC AAT GCA-3' (SEQ ID NO: 13); antisense 5'-GGT CCT TTT CAC CAG CAA GCT-3' (SEQ ID NO: 14), 62°C annealing) MS4A12 transcripts in tumor samples were quantified relative to normal tissues using ΔΔCT calculation. Specificity of PCR reactions was confirmed by cloning and sequencing of amplification products from arbitrarily selected samples.

### Antisera, immunofluorescence and immunochemistry

The polyclonal antiserum was raised against recombinantly expressed fragment of MS4A12 (aa 1-63) and affinity-purified by a custom antibody service (Squarix). Immunohistochemistry was performed on tissue cryosections using the VECTOR NovaRED Substrate Kit (Vector) according to the manufacturer's instructions.

For Western blot analysis 20 µg of total protein extracted from cells lysed with Triton-X was used. Extracts were diluted in reducing sample buffer (Roth, Karlsruhe, Germany), subjected to SDS-PAGE and subsequently electrotransferred onto PVDF membrane (Pall). Immunostaining was performed with antibodies reactive to MS4A12, KRT18 (Abcam) and beta-Actin (Abcam) followed by detection of primary antibody with horseradish-peroxidase conjugated goat anti-mouse and goat anti-rabbit secondary antibodies (Dako).

### siRNA duplexes

Two individual MS4A12 siRNA duplexes (Qiagen) targeting nucleotides 344-363 (siRNA#1, sense 5'-r(UCA UGG UUG GAU UGA UGC A)dTdT-3' (SEQ ID NO: 4), antisense 5'-r(UGC AUC AAU CCA ACC AUG A)dTdC-3' (SEQ ID NO: 5)) and nucleotides 247-266 of the MS4A12 mRNA sequence (NM_017716.1; SEQ ID NO: 1) (siRNA#2, sense 5'-r(CAA CCG GGU CAA GGA AAU A)dTdA-3' (SEQ ID NO: 7), antisense 5'-r(UAU UUC CUU GAC CCG GUU G)dAdC-3' (SEQ ID NO: 8)) were designed. As control a non-silencing siRNA duplex (sense 5'-r(UAA CUG UAU AAU CGA CUA G)dTdT-5' (SEQ ID NO: 9), antisense 5'-r(CUA GUC GAU UAU ACA GUU A)dGdA-3' (SEQ ID NO: 10)) was used. For MS4A12 silencing studies cells were transfected with 10nM siRNA duplex using HiPerFect transfection reagent (Qiagen) according to the manufacturer's instructions.

### Calcium imaging

All buffers contained 125 mM NaCl, 5mM KCl, 1 mM MgCl₂, 20 mM HEPES, and the pH was adjusted to 7.4 with NaOH. CaCl₂ and SrCl₂ were added to the buffer at 1 mM final concentration as indicated. Ca²⁺ free buffer contained 0.2 mM EGTA. Thapsigargin, EGF, and La³⁺ were used at the indicated concentrations. Fluorophore labeling of cells was conducted using FLIPR Calcium 3 Assay Kit (Molecular Devices) according to the manufacturer's instructions. Changes in intracellular calcium were documented using Olympus-IX71 inverted microscope and TILLvisION software (TILL Photonics). Fluorescence values in a field of >50 cells were recorded during the perfusion of various reagents. All assays were done in quadruplicates and repeated twice.

### Cell proliferation analysis

24 h after transfection with siRNA duplexes 1x10⁴ cells were cultured in serum-free medium for 12h. 48 h after medium was supplemented with EGF (50 nM) proliferation was analyzed by measuring the incorporation of BrdU into newly synthesized DNA strands using the DELFIA cell proliferation Kit (Perkin Elmer) according to the manufacturer's instructions on a Wallac Victor² multi-label counter (Perkin Elmer). All assays were done in triplicates and repeated twice.

### Cell cycle analysis

24 h after transfection with siRNA duplexes 1x10⁵ cells were cultured in serum-free medium for 12h. 48 h after medium was supplemented with EGF (50 nM) cells were harvested, EtOH-fixed, and stained with propidiumiodide prior to flowcytometric DNA content analysis. Cells in the different phases of the cell cycle were quantified using FlowJo™ (Tree Star) flowcytometric analysis software. All assays were done in triplicates and repeated twice.

### Cell migration and in vitro invasion assay

Cell motility (chemokinesis) assays were conducted in transwell chambers with 8.0 µm pore membranes (BD Biosciences) with cells cultured in medium with various concentrations of EGF. 24 h after transfection with siRNA duplexes cells were cultured in serum-free medium for 12 h and 4x10⁴ cells were added to the upper chamber. Upper and lower chamber both contained medium with equal concentrations of EGF (50, 10, 2, or 0.4 nM). 24 h later cells that had migrated to the bottom side of the membrane were fixed in ice-cold methanol. Membranes were excised, placed on microscope slides and mounted with Hoechst (Dako) for fluorescence microscopy. Cells in five random visual fields (100x magnification) were counted for each membrane. All experiments were done in triplicates. For in vitro invasion assays the upper chambers were prepared with 100µl of Matrigel (BD Biosciences) diluted to 1mg/ml in serum free medium. Chambers were incubated for 5 h at 37°C for gelling. All assays were done in triplicates and repeated twice.

### Statistics

Correlation analysis of normalized MS4A12 mRNA expression levels in colon cancer samples with tumor stage and grading was done using Kruskal-Wallis non-parametric ANOVA test with GraphPad Prism software package (GraphPad Software). The statistical significance of MS4A12 silencing on proliferation, cell cycle progression, migration and invasion of LoVo cells was calculated using unpaired t-test with GraphPad Prism software package (GraphPad Software) comparing effects in siRNA treated cells with untreated cells.

### Results and discussion

A genome-wide data mining strategy was applied to search for targets for monoclonal antibody therapy of colon cancer. The approach was based on digital cDNA library subtraction to discover differentiation genes with exquisite specificity for colonic epithelial cells structurally resembling the CD20 protein with at least four hydrophobic regions qualifying as transmembrane domains. Such candidates were subsequently tested for conserved expression in cancers derived from this lineage. One of the hits of the computational screen perfectly complying with the used search criteria was MS4A12. This molecule is in fact a member of the MS4A family comprising structurally related cell surface proteins such as CD20 (MS4A1), the high-affinity IgE receptor β chain (FcεRIβ) (MS4A2), and HTm4 (MS4A3) (Ishibashi et al., Gene 264: 87-93, 2001; Liang et al., Immunogenetics 53: 357-68, 2001; Liang et al., Genomics 72: 119-27, 2001). In contrast to the majority of members of the MS4A family, which have been previously shown to be differentiation genes of hematopoetic or lymphatic cell lineages, MS4A12 has not been detected in such cells (Liang et al., Immunogenetics 53: 357-68, 2001; Liang et al., Genomics 72: 119-27, 2001).

To determine which human tissues express MS4A12, a comprehensive set of 27 different normal human tissue specimens from up to five donors per tissue type was profiled by specific endpoint and quantitative real-time RT-PCR. Tissues were classified as negative in case of lack of a visible amplification product in end-point RT-PCR and if not exceeding the cut-off of mean expression in all normal non-colonic tissue samples +3 STDs (99% percentile). MS4A12 expression was found to be confined to normal colon mucosa. In all other normal tissues transcript levels were below the detection limit of standard 35 cycle RT-PCR (Fig. 1a, left) and only trace amounts of MS4A12 transcripts could be detected after 40 cycles of quantitative real-time RT-PCR (Fig. 1a, right) with only 1/5 testis specimens slightly exceeding the expression cut-off (Tab. 1).

**Table 1. Expression of MS4A12 in tissues typed by quantitative real-time RT-PCR. Specimens obtained from two to five different individuals were investigated per normal tissue type. Cut-off for normal tissues was set to mean expression in normal tissues except colon + 3 STDs (relative expression of 10fold above detection limit). In tumor tissues only expression values at least 100fold above detection limit were scored positive.**

| **Normal tissues** | **Positive/tested** |
|---|---|
| Colon | 5/5 |
| Stomach | 0/5 |
| Brain | 0/3 |
| Placenta | 0/5 |
| Liver | 0/5 |
| Pancreas | 0/5 |
| PBMCs rest. | 0/5 |
| PBMCs prolif. | 0/5 |
| Lung | 0/5 |
| Breast | 0/5 |
| Ovary | 0/5 |
| Kidney | 0/5 |
| Testis | 1/5 |
| Spleen | 0/5 |
| Lymph node | 0/5 |
| Endometrium | 0/5 |
| Esophagus | 0/5 |
| Skin | 0/3 |
| Thymus | 0/2 |
| Bladder | 0/3 |
| Muscle | 0/3 |
| Prostate | 0/5 |
| Cervix | 0/5 |
| Adrenal gland | 0/3 |
| Small intestine | 0/4 |
| Tonsil | 0/5 |
| Myocard | 0/2 |
| | |

| **Cancerous tissues** | |
|---|---|
| Colon cancer | 25/40 |
| Breast cancer | 0/20 |
| Lung cancer | 0/20 |
| Prostate cancer | 0/15 |
| Gastric cancer | 0/15 |
| Renal cell cancer | 0/15 |
| Malignant melanoma | 0/10 |
| Hepatocellular cancer | 0/10 |
| Leukemia | 0/10 |
| Head neck cancer | 0/5 |

Profiling of tumor specimens revealed that 25 of 40 (63%) colon cancers expressed MS4A12. In order not to overestimate expression frequency of MS4A12 as measured by qPCR in colon cancer samples, transcript levels of at least 10fold above normal tissue cut-off were classified as positive (Tab. 2).

**Table 2. Characteristics of the colon cancer samples analyzed. Histology, UICC clinical stages, grading (G), and relative expression values of MS4A12. Only tumor samples with expression levels at least 100fold above detection limit were scored positive.**

| **Tissue** | **Histology** | **UICC** | **G** | **Relative expression** | **Positive/ negative** |
|---|---|---|---|---|---|
| #1 | Adenocarcinoma | 1 | 2 | 5 | - |
| #2 | Adenocarcinoma | 1 | 3 | 23779 | + |
| #3 | Adenocarcinoma | 1 | 3 | 12773 | + |
| #4 | Adenocarcinoma | 2 | 2 | 34040 | + |
| #5 | Adenocarcinoma | 2 | 2 | 4 | - |
| #6 | Adenocarcinoma | 2 | 2 | 15432 | + |
| #7 | Adenocarcinoma | 2 | 2 | 12 | - |
| #8 | Adenocarcinoma | 2 | 2 | 15004 | + |
| #9 | Adenocarcinoma | 2 | 2 | 2984 | + |
| #10 | Adenocarcinoma | 2 | 3 | 2038 | + |
| #11 | Adenocarcinoma | 2 | 3 | 20 | - |
| #12 | Adenocarcinoma | 2 | 3 | 15905 | + |
| #13 | Adenocarcinoma | 2 | 3 | 1432 | + |
| #14 | Adenocarcinoma | 2 | 3 | 1662 | + |
| #15 | Adenocarcinoma | 2 | 4 | 4192 | + |
| #16 | Adenocarcinoma | 2 | 4 | 95 | - |
| #17 | Adenocarcinoma | 3 | 2 | 228 | + |
| #18 | Adenocarcinoma | 3 | 2 | 6455 | + |
| #19 | Adenocarcinoma | 3 | 2 | 1573 | + |
| #20 | Adenocarcinoma | 3 | 2 | 9 | - |
| #21 | Adenocarcinoma | 3 | 3 | 25002 | + |
| #22 | Adenocarcinoma | 3 | 3 | 10376 | + |
| #23 | Adenocarcinoma | 3 | 3 | 3 | - |
| #24 | Adenocarcinoma | 3 | 3 | 8938 | + |
| #25 | Adenocarcinoma | 3 | 3 | 11 | - |
| #26 | Adenocarcinoma | 3 | 3 | 2 | - |
| #27 | Adenocarcinoma | 3 | 3 | 8484 | + |
| #28 | Adenocarcinoma | 3 | 4 | 5 | - |
| #29 | Adenocarcinoma | 3 | 4 | 22 | - |
| #30 | Adenocarcinoma | 4 | 2 | 9763 | + |
| #31 | Adenocarcinoma | 4 | 2 | 3 | - |
| #32 | Adenocarcinoma | 4 | 2 | 7593 | + |
| #33 | Adenocarcinoma | 4 | 2 | 15 | - |
| #34 | Adenocarcinoma | 4 | 3 | 3849 | + |
| #35 | Adenocarcinoma | 4 | 3 | 14 | - |
| #36 | Adenocarcinoma | 4 | 3 | 1922 | + |
| #37 | Adenocarcinoma | 4 | 3 | 8654 | + |
| #38 | Adenocarcinoma | 4 | 3 | 158371 | + |
| #39 | Adenocarcinoma | 4 | 4 | 1398 | + |
| #40 | Adenocarcinoma | 4 | 4 | 2 | - |

Within the sample population expression levels of MS4A12 did not correlate with tumor stage or tumor grade (p=0.68 and p=0.36, Kruskal-Wallis). MS4A12 was not detected in any other human tumor type including breast cancer, lung cancer, prostate cancer, gastric cancer, renal cell cancer, malignant melanoma, hepatocellular cancer, leukemia, head neck cancer (Tab. 1).

For analysis of protein expression, an affinity purified polyclonal rabbit antibody (anti-MS4A12/N-term) raised against a recombinantly expressed polypeptide corresponding to the N-terminal 63 amino acids of MS4A12 (NP_060186) was used. To verify specificity of the antibody gene silencing of MS4A12 by small interfering RNA (siRNA) was used. Transfection of MS4A12 expressing colon cancer cell line LoVo with two individual MS4A12 specific siRNA duplexes resulted in stable and reproducible reduction of constitutive MS4A12 mRNA expression by 90%, whereas scrambled non-silencing control duplexes had no effect (Fig. 6). Consistent with this observation, the -35 kDa band, detected in accordance with the predicted size of MS4A12 in lysates of LOVO cells in Western blot, disappeared completely (Fig. 1b). This proved both robust knock down of MS4A12 protein expression as well as specificity of the antibody. Western Blot staining of MS4A12 protein in primary human tissue samples with anti-MS4A12/N-term confirmed MS4A12 protein expression in colon cancer specimens in levels comparable to normal colon mucosa as the only expressing normal tissue, but not in tissues typed negative by RT-PCR. Normalization to cytokeratin-18 (KRT18) as a marker for epithelial cells ruled out that high MS4A12 protein levels in colon cancers were observed as a result of a higher portion of epithelial cells in the specimen.

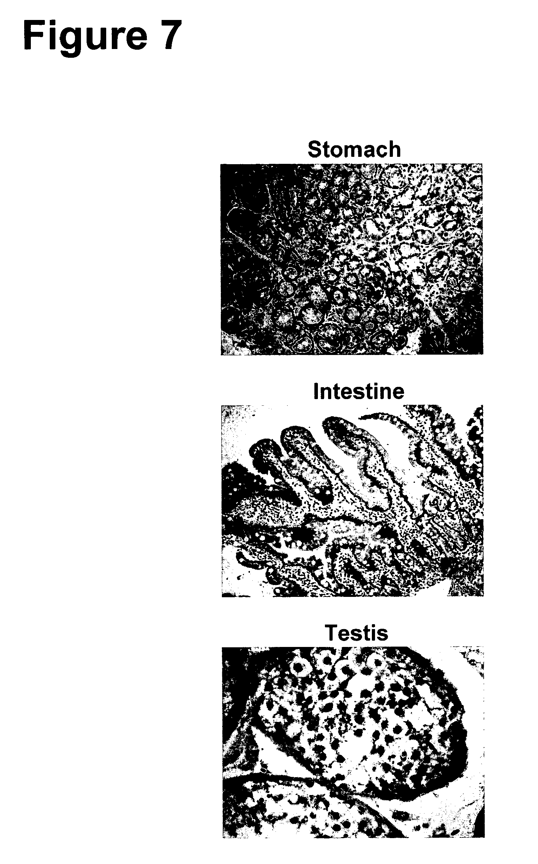

Immunohistochemistry with anti-MS4A12/N-term antibody on normal colon mucosa revealed a characteristic and distinct apical membrane staining of the epithelial cells of normal colonic crypts (Fig. 1c). In sections obtained from other gastrointestinal tissues and testis MS4A12 staining was not detectable (Fig. 7), excluding that smaller subpopulations present within these composite organ tissues do express this protein. In colon cancer only the neoplastic cell population was stained, whereas adjacent stromal and non-neoplastic epithelial cells were negative. Though additional cytoplasmic immunostaining was observed, staining of tumor cells was clearly accentuated at the plasma membrane, substantiating predictions that MS4A12 is a cell surface protein (Liang et al., Genomics 72: 119-27, 2001). In the pilot set of stained colon cancer samples, tumors with homogenous and strong expression, as well as specimens with weaker and more heterogeneous expression were observed (Fig. 1c).

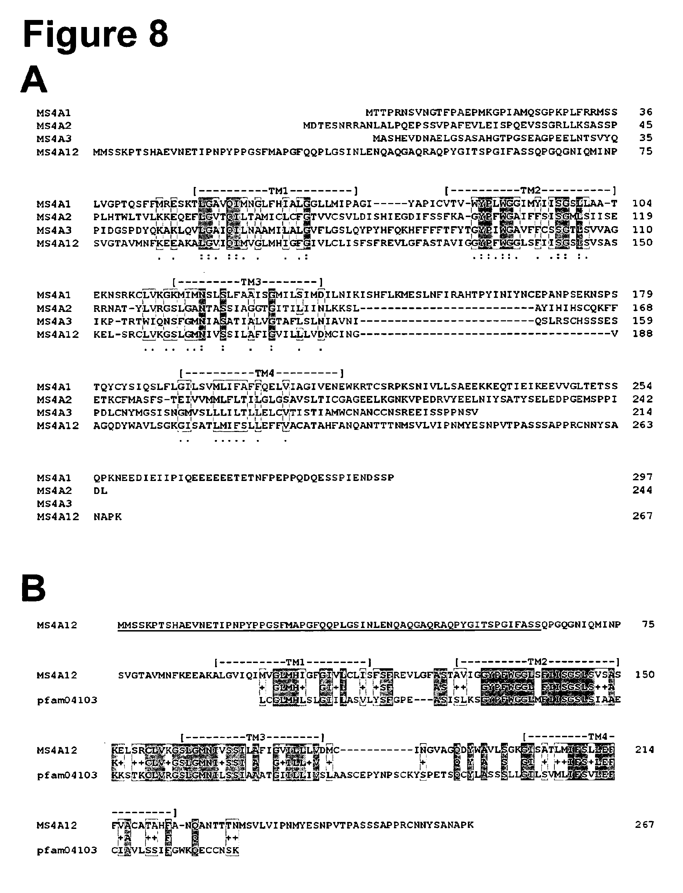

Membrane topology of CD20 as prototypic member of the MS4A family has been extensively characterized. CD20 is a 297 aa protein with cytoplasmic amino- and carboxy-terminal ends and four transmembrane domains forming two extracellular loops (Cartron et al., Blood 104: 2635-42, 2004). As there is a considerable sequence identity among MS4A family members (Fig. 8a), it has been predicted that other MS4A proteins share the surface localization and peculiar topology of CD20 (Liang et al., Genomics 72: 119-27, 2001). Alignment of the MS4A12 protein sequence with the CD20 domain (pfam04103) showed a 43% sequence similarity on the amino acid level (Fig. 8b). Analogous to reports for other MS4A12 family members, the highest degree of identity was found in the first three transmembrane stretches (Liang et al., Genomics 72: 119-27, 2001) and a tetraspan membrane topology was predicted (Fig. 2a). Cell membrane localization was experimentally confirmed by immunofluorescence microscopy of CHO cells transfected with eGFP-tagged MS4A12 constructs (Fig. 2b). Further support was provided by the distinct plasma membrane staining of LoVo colon cancer cells constitutively expressing MS4A12 by anti-MS4A12/N-term antibody, which was lost upon siRNA-induced knock down of MS4A12 (Fig. 2c). To verify the predicted topology, CHO cells were transfected with MS4A12 constructs tagged either N- or C-terminally with eGFP and stained with anti-eGFP antibodies after PFA-fixation. Both N-and C-terminally localized eGFP was only detectable by flowcytometric analysis after permeabilization of transfected cells with saponin (Fig. 2d), but not under conditions impairing penetration of the antibody into the cells. In summary, these data support cell surface localization of MS4A12 as well as the predicted topology with intracellular localization of the N- and C-terminus.

As CD20 has been shown to possess store-operated Ca²⁺ channel (SOCC) activity (Li et al., J. Biol. Chem. 278: 42427-34, 2003), it was hypothesized that MS4A12 may also be involved in capacitative calcium entry. CHO cells transfected either with an MS4A12 encoding plasmid or with the empty vector as control were treated with Thapsigargin (Tg), a specific inhibitor of the endoplasmic reticular Ca²⁺-ATPase (SERCA) to deplete intracellular calcium stores (Thastrup et al., Proc. Natl. Acad. Sci. U S A 87: 2466-70, 1990). In addition to Ca²⁺ entry also Sr²⁺ influx was tested, as Sr²⁺ entry can potentially distinguish between endogenous and exogenously expressed SOC channels (Li et al., J. Biol. Chem. 278: 42427-34, 2003; Ma et al., Science 287: 1647-51, 2000). Baseline Ca²⁺ levels as measured by fluorescence detection of a Ca²⁺-sensitive dye were identical in control and in MS4A12 expressing CHO cells (Fig. 3a). As expected, Tg sharply increased [Ca²⁺]_{c} in the absence of extracellular Ca²⁺ in both cell lines. [Ca²⁺]_{c} gradually returned to base line levels, most likely due to extrusion mechanisms at the plasma membrane and by uptake into internal organelles (Bootman et al., Semin. Cell Dev. Biol. 12: 3-10, 2001). Subsequent perfusion of cells with Sr²⁺ induced a considerable fluorescence increase in MS4A12-transfected cells, suggesting that influx of Sr²⁺ occurred in response to store depletion. Vector-transfected cells did not respond to Sr²⁺, indicating that endogenous SOC channels are impermeable to Sr²⁺ under the experimental conditions. No Sr²⁺ entry occurred prior to store depletion, indicating that MS4A12 expression does not confer constitutive permeability to Sr²⁺ in CHO cells. After Sr²⁺ was withdrawn and fluorescence values had returned to baseline, Ca²⁺ was introduced into the perfusion chamber. Influx of Ca²⁺ into vector-transfected cells indicated that these cells maintained a normal Ca²⁺ entry pathway after Tg treatment. Calcium influx into MS4A12-transfected cells was significantly increased over that observed in vector-transfected cells. Pretreatment of cells with the known SOC channel blocker La³⁺ resulted in marked reduction of Ca²⁺ influx into MS4A12-transfected cells, which in control cells was not detectable at all (Fig. 3a). Sr²⁺ entry in both cell lines was completely abolished. In summary, these data demonstrate that MS4A12 has the ability to form and/or organize a SOC entry pathway.

Ca²⁺ flux is the fastest response following activation of receptor tyrosine kinases (Munaron, Int. J. Mol. Med. 10: 671-6, 2002; Patterson et al., Trends Biochem. Sci. 30: 688-97, 2005). Binding of growth factors, such as EGF to their receptors on the plasma membrane leads to activation of phospholipase C γ (PLCγ) that hydrolyzes membrane lipids to produce inositol 1,4,5-trisphophate (IP₃). IP₃ binds to the IP₃ receptor located on the membrane of the ER and stimulates Ca²⁺ release into the cytoplasm. This, in turn, is associated with SOC entry. For functions, for which prolongation of the Ca signal plays an important role such as cell growth, SOC entry is a major component of calcium signaling (Berridge et al., Nature 395: 645-8, 1998; Berridge et al., Nat. Rev. Mol. Cell Biol. 1: 11-21, 2000). To assess the role of MS4A12 in calcium entry in response to growth factor signaling, we used MS4A12 positive LoVo colon cancer cells, which express high levels of epidermal growth factor receptor (EGFR) (Pai et al., Nat. Med. 8: 289-93, 2002). Treatment of LoVo cells with epidermal growth factor (EGF) has been shown to induce SOC entry in a PLCγ dependent manner (Munaron, Int. J. Mol. Med. 10: 671-6, 2002; Kokoska et al., J. Gastrointest. Surg. 4: 150-61, 2000; Kokoska et al., J. Surg. Res. 88: 97-103, 2000; Ma et al., J. Am. Soc. Nephrol. 12: 47-53, 2001). LoVo cells transfected with MS4A12-specific siRNAs or scrambled control siRNA were treated with Tg and two different concentrations of EGF. Again no difference in baseline fluorescence was detected. In both cell lines Tg as well as EGF both sharply increased [Ca²⁺]_{c} in the absence of extracellular Ca²⁺. SOC entry, however, was significantly impaired in cells with MS4A12 expression knock down, but not in cells transfected with control siRNA (Fig. 3b, c). Pretreatment of both cell lines with La³⁺ inhibited SOC entry completely. Noteworthy, Ca²⁺ entry in cells treated with low concentrations of EGF (2nM) was nearly completely abolished by MS4A12 silencing. According to this data, SOC entry in LoVo cells is at least partially mediated by MS4A12. The Ca²⁺ entry remaining after MS4A12 silencing indicates the presence of other SOCCs in this cell system.

Growth factor-induced signaling is often organized in an oscillatory pattern (Munaron, Int. J. Mol. Med. 10: 671-6, 2002). Repetitive calcium spikes require both the entry of external calcium via SOCCs and its release from internal stores. This results in activation of immediate early genes responsible for inducing resting cells (G0) to re-enter the cell cycle (Berridge, Bioessays 17: 491-500, 1995). Most interestingly, in clinical trials targeting SOC entry by Ca²⁺ influx inhibitors, tumor growth inhibition of certain cancers is observed (Kohn et al., Cancer Res. 56: 569-73, 1996; Wasilenko et al., Int. J. Cancer 68: 259-64, 1996). Therefore, the impact of MS4A12 expression on the proliferation of LoVo cells in response to EGF was assessed. A significant reduction of growth factor induced cell proliferation was observed in cells with siRNA mediated silencing of MS4A12 as compared to control cells (Fig. 4a). Cell cycle analysis revealed a distinct G1/S arrest in cells transfected with MS4A12 siRNAs as the underlying cause for the proliferation block (Fig. 4b), whereas vitality of the cells was not affected.

Another important cellular function tightly regulated by the availability of free cytoplasmic Ca²⁺ is growth factor-induced cell motility and migration (Berridge et al., Nat. Rev. Mol. Cell Biol. 1: 11-21, 2000). The increase in free cytoplasmic Ca²⁺ by InsP3 mediated release of Ca²⁺ from intracellular pools results in activation of the actin cytoskeleton in the immediate submembrane region (Feldner et al., Exp. Cell Res. 272: 93-108, 2002), which is elementary for cell migration. The impact of MS4A12 on growth factor mediated motility of LoVo cells was investigated in transwell migration assays. Baseline motility (chemokinesis) was assessed by adding various concentrations of EGF to both the upper and lower chamber of the system. Motility was significantly reduced upon knock down of MS4A12 (Fig 5a). Chemoinvasive activity of cells, as well, as measured by their ability to break through a barrier of Matrigel to migrate along EGF gradients was profoundly affected and substantially impaired by MS4A12 knock down (Fig. 5b). Intriguingly, the impact of MS4A12 silencing on both cellular functions was particularly pronounced in cells exposed to low concentrations of EGF. Enhanced Ca²⁺ flux mediated by expression of MS4A12 lowers the threshold concentration of EGF required for the activation of the EGF receptor (EGFR) pathway.

In summary, the first functional characterization of MS4A12 is presented. It is revealed that MS4A12 is a component of SOC entry and modulates EGFR signaling in colon cancer cells. The strict restriction of expression to colonocytes implies a specialized role of MS4A12 in this tissue type. This is further supported by its involvement in modulation of EGFR signaling, which has a pivotal role in the development and maintenance of intestinal epithelial continuity and is involved in control of a variety of epithelial cell properties, such as growth, healing, adhesion and cell migration (Carpenter et al., Annu. Rev. Biochem. 48: 193-216, 1979; Jones et al., Front. Biosci. 4: 303-9, 1999; Lacy, J. Clin. Gastroenterol. 10: 72-7, 1988; Tarnawski et al., J. Clin. Gastroenterol. 27: 12-20, 1998). Other growth factors and peptides involved in these functions (e.g. TGFα, intestinal trefoil factor) all act, at least partially, via the EGFR signaling pathway (Giraud, Am. J. Physiol. Gastrointest. Liver Physiol. 278: 501-6, 2000). Whereas most cognate receptors are on the epithelial basolateral surface, cytoprotective trefoil peptides are secreted onto the apical surface of the mucosa (Chinery et al., Br. J. Pharmacol. 115: 77-80, 1995; Podolsky, J. Gastroenterol. 32: 122-6, 1997). It is tempting to speculate that the spatially restricted expression of MS4A12 in the apical epithelial monolayer might render these cells more sensitive to the cytoprotective action of such factors. MS4A12 expression is maintained upon malignant transformation and is detectable in a significant portion of colon cancers. As modulator of EGFR signaling, which is the dominant tumor promoting factor in colon cancer, it is linked to features contributing to the tumor phenotype such as survival, proliferation and motility as supported by the siRNA data described above. The observation that loss of MS4A12 impairs such EGFR-dependent cell functions, whereas expression of MS4A12 lowers the threshold for EGF-triggered Ca²⁺ entry has clinical impact. MS4A12 expression status of colon cancer patients affects sensitivity of their cancer cells to EGF and thereby their prognosis. Moreover, response of patients to treatment with antibodies against EGFR, such as Erbitux, is expected to correlate with MS4A12 status of their tumors.

Moreover, several findings qualify MS4A12 as a novel target candidate for therapeutic antibodies.

First, being a lineage specific marker highly selective for colonic epithelia, MS4A12 is absent from other toxicity-relevant normal tissues. Despite their expression in normal colonic mucosa, mAbs targeting differentiation antigens in colon cancer can be safely administered without inducing toxic side effects in the normal mucosa (Scott et al., Clin. Cancer Res. 11: 4810-7, 2005), most likely resulting from the high colonocyte turnover (Lipkin et al., Gastroenterology 45: 721-9, 1963), counteracting accumulation of the antibody in normal mucosa. The narrow spatial expression pattern of MS4A12 in the most apical region of the normal mucosa further adds to the safety of MS4A12 targeting antibodies.

Second, MS4A12 expression is maintained in a significant portion of colon cancers. Large scale analyses of colon cancer specimens by immunohistochemistry are underway to further assess the prevalence of MS4A12 protein expression and homogeneity of this target candidate in individual tumor specimens.

Third, MS4A12 is a surface molecule and is therefore potentially drugable by antibodies. Sequence similarity to other MS4A family members and our experimental data strongly suggest that MS4A12, like CD20 is embedded into the plasmamembrane with two extracellular loops. The consequence of this particular topology is that (i) shedding of the antigen is prevented, (ii) binding mAbs are highly concentrated in small areas of the plasma membrane, and that (iii) there is a short distance between the target epitope and the cell surface. These features have been reported to enhance the ability of mAbs to induce efficient recruitment of cytotoxic immune effector mechanisms (Bindon et al., Eur. J. Immunol. 18: 1507-14, 1988; Borsos et al., Science 150: 505-6, 1965; Golan et al., J. Immunol. 129: 445-7, 1982). The structural similarity to CD20 makes it likely that antibodies exhibiting recruitment of complement and FcR-positive effector cells can be engineered. Its link to EGFR signaling positions MS4A12 in a pathway which is already being addressed by a variety of mAbs and small compounds. Many of these therapies are approved or are currently in advanced clinical development which adds to its attractiveness as drug target.

The data presented herein demonstrate in particular that MS4A12 bears a therapeutic potential as antibody target in colon cancer.

## Claims

1. An agent which reduces or inhibits expression or activity of MS4A12 in a cell.

2. The agent of claim 1, wherein said reduction or inhibition of expression or activity of MS4A12 in a cell reduces or inhibits growth, proliferation, viability, cell cycle progression, migration, chemotaxis and/or invasion of said cell.

3. The agent of claim 1 or 2, wherein the activity of MS4A12 in a cell is increasing or enabling responsiveness of said cell towards EGF, wherein the increase in responsiveness of the cell towards EGF preferably is an increase of EGF-induced growth, EGF-induced proliferation, EGF-induced cell cycle progression, EGF-induced migration, EGF-induced chemotaxis and/or EGF-induced invasion of said cell.

4. The agent of any one of claims 1 to 3 which is selected from the group consisting of
(i) an siRNA specifically targeting MS4A12 mRNA,
(ii) an antisense nucleic acid capable of hybridizing selectively with a nucleic acid coding for MS4A12,
(iii) an antibody capable of selectively binding to MS4A12, and
(iv) a nucleic acid molecule capable of expressing the siRNA of (i), the antisense nucleic acid of (ii) or the antibody of (iii).

5. The agent of any one of claims 1 to 4, wherein the activity of MS4A12 in a cell involves increasing or enabling store-operated Ca²⁺ entry in said cell.

6. A composition comprising the agent of any one of claims 1 to 5.

7. The composition of claim 6, further comprising an agent which reduces or inhibits expression or activity of an EGF receptor in a cell.

8. The composition of claim 6 or 7 which is a pharmaceutical composition.

9. The agent of any one of claims 1 to 5 or the composition of claim 8 for use in medicine.

10. The agent of any one of claims 1 to 5 or the composition of claim 8 for the treatment of cancer or cancer metastasis.

11. A method for reducing or inhibiting responsiveness of a cell towards EGF comprising contacting said cell with the agent of any one of claims 1 to 5.

12. The method of claim 11, further comprising contacting said cell with an agent which reduces or inhibits expression or activity of an EGF receptor in a cell.

13. A method for treating cancer or cancer metastasis, preferably colon cancer or colon cancer metastasis, in a patient comprising administering the agent of any one of claims 1 to 5 to the patient.

14. The method of claim 13, further comprising administering an agent which reduces or inhibits expression or activity of an EGF receptor in a cell to the patient.

15. The method of claim 13 or 14, wherein the composition reduces or inhibits tumor growth, tumor invasion and/or tumor metastasis in the patient.

16. The method of any one of claims 13 to 15, wherein at least a part of the cancer cells are responsive towards EGF.

17. A method for determining the responsiveness of a cell towards EGF comprising determining the level of expression and/or activity of MS4A12 in the cell.

18. The method of claim 17, wherein the determination of the level of expression of MS4A12 in the cell comprises determining the amount of MS4A12 mRNA and/or MS4A12 protein in said cell and/or the determination of the level of activity of MS4A12 in the cell comprises determining store-operated Ca²⁺ entry in said cell, wherein the store-operated Ca²⁺ entry preferably is determined in the presence and absence of an agent which reduces or inhibits expression or activity of MS4A12 in a cell.

19. The method of claim 18, wherein the determination of store-operated Ca²⁺ entry in the cell comprises determining the amount and/or time course of Ca²⁺ or Sr²⁺ entry into the cell, wherein the amount and/or time course of Ca²⁺ or Sr²⁺ entry into the cell preferably is determined after depletion of an intracellular Ca²⁺ store.

20. The method of any one of claims 17 to 19, wherein the cell is a tumor cell and wherein the method preferably is for determining the responsiveness of a tumor towards EGF and/or the responsiveness of a tumor towards anti-EGF tumor therapy.

21. A method for increasing the responsiveness of a cell towards EGF comprising increasing expression or activity of MS4A12 in said cell.


SEQUENCE LISTING

<110>  Johannes Gutenberg-Universität Mainz

<120>  Methods involving MS4A12 and agents targeting MS4A12 for therapy,
        diagnosis and testing

<130>  342-43

<160>  20

<170>  PatentIn version 3.3

<210>  1
<211>  1182
<212>  DNA
<213>  Homo sapiens

<400>  1
acacaggttg gagcagagaa agaggaaaca tagaggtgcc aaaggaacaa agacataatg      60

atgtcatcca agccaacaag ccatgctgaa gtaaatgaaa ccatacccaa cccttaccca     120

ccaggcagct ttatggctcc tggatttcaa cagcctctgg gttcaatcaa cttagaaaac     180

caagctcagg gtgctcagcg tgctcagccc tacggcatca catctccggg aatctttgct     240

agcagtcaac cgggtcaagg aaatatacaa atgataaatc caagtgtggg aacagcagta     300

atgaacttta aagaagaagc aaaggcacta ggggtgatcc agatcatggt tggattgatg     360

cacattggtt ttggaattgt tttgtgttta atatccttct cttttagaga agtattaggt     420

tttgcctcta ctgctgttat tggtggatac ccattctggg gtggcctttc ttttattatc     480

tctggctctc tctctgtgtc agcatccaag gagctttccc gttgtctggt gaaaggcagc     540

ctgggaatga acattgttag ttctatcttg gccttcattg gagtgattct gctgctggtg     600

gatatgtgca tcaatggggt agctggccaa gactactggg ccgtgctttc tggaaaaggc     660

atttcagcca cgctgatgat cttctccctc ttggagttct tcgtagcttg tgccacagcc     720

cattttgcca accaagcaaa caccacaacc aatatgtctg tcctggttat tccaaatatg     780

tatgaaagca accctgtgac accagcgtct tcttcagctc ctcccagatg caacaactac     840

tcagctaatg cccctaaata gtaaaagaaa aaggggtatc agtctaatct catggagaaa     900

aactacttgc aaaaacttct taagaagatg tcttttattg tctacaatga tttctagtct     960

ttaaaaactg tgtttgagat ttgtttttag gttggtcgct aatgatggct gtatctccct    1020

tcactgtctc ttcctacatt accactacta catgctggca aaggtgaagg atcagaggac    1080

tgaaaaatga ttctgcaact ctcttaaagt tagaaatgtt tctgttcata ttactttttc    1140

cttaataaaa tgtcattaga aacaaaaaaa aaaaaaaaaa aa                       1182

<210>  2
<211>  267
<212>  PRT
<213>  Homo sapiens

<400>  2

Met Met Ser Ser Lys Pro Thr Ser His Ala Glu Val Asn Glu Thr Ile

1                   5                   10                  15

Pro Asn Pro Tyr Pro Pro Gly Ser Phe Met Ala Pro Gly Phe Gln Gln
            20                  25                  30

Pro Leu Gly Ser Ile Asn Leu Glu Asn Gln Ala Gln Gly Ala Gln Arg
        35                  40                  45

Ala Gln Pro Tyr Gly Ile Thr Ser Pro Gly Ile Phe Ala Ser Ser Gln
    50                  55                  60

Pro Gly Gln Gly Asn Ile Gln Met Ile Asn Pro Ser Val Gly Thr Ala
65                  70                  75                  80

Val Met Asn Phe Lys Glu Glu Ala Lys Ala Leu Gly Val Ile Gln Ile
                85                  90                  95

Met Val Gly Leu Met His Ile Gly Phe Gly Ile Val Leu Cys Leu Ile
            100                 105                 110

Ser Phe Ser Phe Arg Glu Val Leu Gly Phe Ala Ser Thr Ala Val Ile
        115                 120                 125

Gly Gly Tyr Pro Phe Trp Gly Gly Leu Ser Phe Ile Ile Ser Gly Ser
    130                 135                 140

Leu Ser Val Ser Ala Ser Lys Glu Leu Ser Arg Cys Leu Val Lys Gly
145                 150                 155                 160

Ser Leu Gly Met Asn Ile Val Ser Ser Ile Leu Ala Phe Ile Gly Val
                165                 170                 175

Ile Leu Leu Leu Val Asp Met Cys Ile Asn Gly Val Ala Gly Gln Asp
            180                 185                 190

Tyr Trp Ala Val Leu Ser Gly Lys Gly Ile Ser Ala Thr Leu Met Ile
        195                 200                 205

Phe Ser Leu Leu Glu Phe Phe Val Ala Cys Ala Thr Ala His Phe Ala
    210                 215                 220

Asn Gln Ala Asn Thr Thr Thr Asn Met Ser Val Leu Val Ile Pro Asn
225                 230                 235                 240

Met Tyr Glu Ser Asn Pro Val Thr Pro Ala Ser Ser Ser Ala Pro Pro
                245                 250                 255

Arg Cys Asn Asn Tyr Ser Ala Asn Ala Pro Lys
            260                 265

<210>  3

<211>  21
<212>  DNA
<213>  Artificial sequence

<220>
<223>  Target sequence

<400>  3
gatcatggtt ggattgatgc a                                                21


<210>  4
<211>  21
<212>  DNA
<213>  Artificial sequence

<220>
<223>  siRNA sense strand


<220>
<221>  misc_feature
<222>  (1)..(19)
<223>  ribonucleotide bases

<400>  4
ucaugguugg auugaugcat t                                                21


<210>  5
<211>  21
<212>  DNA
<213>  Artificial sequence

<220>
<223>  siRNA antisense strand


<220>
<221>  misc_feature
<222>  (1)..(19)
<223>  ribonucleotide bases

<400>  5
ugcaucaauc caaccaugat c                                                21


<210>  6
<211>  21
<212>  DNA
<213>  Artificial sequence

<220>
<223>  Target sequence

<400>  6
gtcaaccggg tcaaggaaat a                                                21


<210>  7
<211>  21
<212>  DNA
<213>  Artificial sequence

<220>
<223>  siRNA sense strand

<220>
<221> misc_feature
<222> (1)..(19)
<223> ribonucleotide bases

<400> 7
caaccggguc aaggaaauat a 21


<210> 8
<211> 21
<212> DNA
<213> Artificial sequence

<220>
<223> siRNA antisense strand


<220>
<221> misc_feature
<222> (1)..(19)
<223> ribonucleotide bases

<400> 8
uauuuccuug acccgguuga c 21


<210> 9
<211> 21
<212> DNA
<213> Artificial sequence

<220>
<223> siRNA sense strand


<220>
<221> misc_feature
<222> (1)..(19)
<223> ribonucleotide bases

<400> 9
uaacuguaua aucgacuagt t 21


<210> 10
<211> 21
<212> DNA
<213> Artificial sequence

<220>
<223> siRNA antisense strand


<220>
<221> misc_feature
<222> (1)..(19)
<223> ribonucleotide bases

<400> 10
cuagucgauu auacaguuag a 21


<210> 11
<211> 21
<212> DNA
<213> Artificial sequence

<220>
<223>  Oligonucleotide

<400>  11
gagctttccc gttgtctggt g                                                21


<210>  12
<211>  21
<212>  DNA
<213>  Artificial sequence

<220>
<223>  Oligonucleotide

<400>  12
gctgaagaag acgctggtgt c                                                21


<210>  13
<211>  21
<212>  DNA
<213>  Artificial sequence

<220>
<223>  Oligonucleotide

<400>  13
tgacactggc aaaacaatgc a                                                21


<210>  14
<211>  21
<212>  DNA
<213>  Artificial sequence

<220>
<223>  Oligonucleotide

<400>  14
ggtccttttc accagcaagc t                                                21


<210>  15
<211>  23
<212>  DNA
<213>  Artificial sequence

<220>
<223>  Oligonucleotide

<400>  15
cgtgagcgct tcgagatgtt ccg                                              23


<210>  16
<211>  23
<212>  DNA
<213>  Artificial sequence

<220>
<223>  Oligonucleotide

<400>  16
cctaaccagc tgcccaactg tag                                              23


<210>  17

<211>  297
<212>  PRT
<213>  Homo sapiens

<400>  17

Met Thr Thr Pro Arg Asn Ser Val Asn Gly Thr Phe Pro Ala Glu Pro
1               5                   10                  15

Met Lys Gly Pro Ile Ala Met Gln Ser Gly Pro Lys Pro Leu Phe Arg
            20                  25                  30

Arg Met Ser Ser Leu Val Gly Pro Thr Gln Ser Phe Phe Met Arg Glu
        35                  40                  45

Ser Lys Thr Leu Gly Ala Val Gln Ile Met Asn Gly Leu Phe His Ile
    50                  55                  60

Ala Leu Gly Gly Leu Leu Met Ile Pro Ala Gly Ile Tyr Ala Pro Ile
65                  70                  75                  80

Cys Val Thr Val Trp Tyr Pro Leu Trp Gly Gly Ile Met Tyr Ile Ile
                85                  90                  95

Ser Gly Ser Leu Leu Ala Ala Thr Glu Lys Asn Ser Arg Lys Cys Leu
            100                 105                 110

Val Lys Gly Lys Met Ile Met Asn Ser Leu Ser Leu Phe Ala Ala Ile
        115                 120                 125

Ser Gly Met Ile Leu Ser Ile Met Asp Ile Leu Asn Ile Lys Ile Ser
    130                 135                 140

His Phe Leu Lys Met Glu Ser Leu Asn Phe Ile Arg Ala His Thr Pro
145                 150                 155                 160

Tyr Ile Asn Ile Tyr Asn Cys Glu Pro Ala Asn Pro Ser Glu Lys Asn
                165                 170                 175

Ser Pro Ser Thr Gln Tyr Cys Tyr Ser Ile Gln Ser Leu Phe Leu Gly
            180                 185                 190

Ile Leu Ser Val Met Leu Ile Phe Ala Phe Phe Gln Glu Leu Val Ile
        195                 200                 205

Ala Gly Ile Val Glu Asn Glu Trp Lys Arg Thr Cys Ser Arg Pro Lys
    210                 215                 220

Ser Asn Ile Val Leu Leu Ser Ala Glu Glu Lys Lys Glu Gln Thr Ile
225                 230                 235                 240

Glu Ile Lys Glu Glu Val Val Gly Leu Thr Glu Thr Ser Ser Gln Pro
                245                 250                 255

Lys Asn Glu Glu Asp Ile Glu Ile Ile Pro Ile Gln Glu Glu Glu Glu
            260                 265                 270

Glu Glu Thr Glu Thr Asn Phe Pro Glu Pro Pro Gln Asp Gln Glu Ser
        275                 280                 285

Ser Pro Ile Glu Asn Asp Ser Ser Pro
    290                 295

<210>  18
<211>  244
<212>  PRT
<213>  Homo sapiens

<400>  18

Met Asp Thr Glu Ser Asn Arg Arg Ala Asn Leu Ala Leu Pro Gln Glu
1               5                   10                  15

Pro Ser Ser Val Pro Ala Phe Glu Val Leu Glu Ile Ser Pro Gln Glu
            20                  25                  30

Val Ser Ser Gly Arg Leu Leu Lys Ser Ala Ser Ser Pro Pro Leu His
        35                  40                  45

Thr Trp Leu Thr Val Leu Lys Lys Glu Gln Glu Phe Leu Gly Val Thr
    50                  55                  60

Gln Ile Leu Thr Ala Met Ile Cys Leu Cys Phe Gly Thr Val Val Cys
65                  70                  75                  80

Ser Val Leu Asp Ile Ser His Ile Glu Gly Asp Ile Phe Ser Ser Phe
                85                  90                  95

Lys Ala Gly Tyr Pro Phe Trp Gly Ala Ile Phe Phe Ser Ile Ser Gly
            100                 105                 110

Met Leu Ser Ile Ile Ser Glu Arg Arg Asn Ala Thr Tyr Leu Val Arg
        115                 120                 125

Gly Ser Leu Gly Ala Asn Thr Ala Ser Ser Ile Ala Gly Gly Thr Gly
    130                 135                 140

Ile Thr Ile Leu Ile Ile Asn Leu Lys Lys Ser Leu Ala Tyr Ile His
145                 150                 155                 160

Ile His Ser Cys Gln Lys Phe Phe Glu Thr Lys Cys Phe Met Ala Ser
                165                 170                 175

Phe Ser Thr Glu Ile Val Val Met Met Leu Phe Leu Thr Ile Leu Gly
            180                 185                 190

Leu Gly Ser Ala Val Ser Leu Thr Ile Cys Gly Ala Gly Glu Glu Leu
        195                 200                 205

Lys Gly Asn Lys Val Pro Glu Asp Arg Val Tyr Glu Glu Leu Asn Ile
    210                 215                 220

Tyr Ser Ala Thr Tyr Ser Glu Leu Glu Asp Pro Gly Glu Met Ser Pro
225                 230                 235                 240

Pro Ile Asp Leu

<210>  19
<211>  214
<212>  PRT
<213>  Homo sapiens

<400>  19

Met Ala Ser His Glu Val Asp Asn Ala Glu Leu Gly Ser Ala Ser Ala
1               5                   10                  15

His Gly Thr Pro Gly Ser Glu Ala Gly Pro Glu Glu Leu Asn Thr Ser
            20                  25                  30

Val Tyr Gln Pro Ile Asp Gly Ser Pro Asp Tyr Gln Lys Ala Lys Leu
        35                  40                  45

Gln Val Leu Gly Ala Ile Gln Ile Leu Asn Ala Ala Met Ile Leu Ala
    50                  55                  60

Leu Gly Val Phe Leu Gly Ser Leu Gln Tyr Pro Tyr His Phe Gln Lys
65                  70                  75                  80

His Phe Phe Phe Phe Thr Phe Tyr Thr Gly Tyr Pro Ile Trp Gly Ala
                85                  90                  95

Val Phe Phe Cys Ser Ser Gly Thr Leu Ser Val Val Ala Gly Ile Lys
            100                 105                 110

Pro Thr Arg Thr Trp Ile Gln Asn Ser Phe Gly Met Asn Ile Ala Ser
        115                 120                 125

Ala Thr Ile Ala Leu Val Gly Thr Ala Phe Leu Ser Leu Asn Ile Ala
    130                 135                 140

Val Asn Ile Gln Ser Leu Arg Ser Cys His Ser Ser Ser Glu Ser Pro
145                 150                 155                 160

Asp Leu Cys Asn Tyr Met Gly Ser Ile Ser Asn Gly Met Val Ser Leu
                165                 170                 175

Leu Leu Ile Leu Thr Leu Leu Glu Leu Cys Val Thr Ile Ser Thr Ile
            180                 185                 190

Ala Met Trp Cys Asn Ala Asn Cys Cys Asn Ser Arg Glu Glu Ile Ser
        195                 200                 205

Ser Pro Pro Asn Ser Val
    210

<210>  20
<211>  144
<212>  PRT
<213>  Homo sapiens

<400>  20

Leu Cys Gly Leu Met His Leu Ser Leu Gly Ile Ile Leu Ala Ser Val
1               5                   10                  15

Leu Tyr Ser Phe Pro Glu Ala Ser Ile Ser Leu Lys Ser Gly Tyr Pro
            20                  25                  30

Phe Trp Gly Gly Leu Met Phe Ile Ile Ser Gly Ser Leu Ser Ile Ala
        35                  40                  45

Ala Glu Lys Lys Ser Thr Lys Cys Leu Val Arg Gly Ser Leu Gly Met
    50                  55                  60

Asn Ile Leu Ser Ser Ile Ala Ala Ala Thr Gly Ile Ile Leu Leu Ile
65                  70                  75                  80

Val Ser Leu Ala Ala Ser Cys Glu Pro Tyr Asn Pro Ser Cys Lys Tyr
                85                  90                  95

Ser Pro Glu Thr Ser Gln Cys Tyr Leu Ala Ser Ser Ser Leu Leu Gly
            100                 105                 110

Ile Leu Ser Val Met Leu Ile Phe Ser Val Leu Glu Phe Cys Ile Ala
        115                 120                 125

Val Leu Ser Ser Ile Phe Gly Trp Lys Gln Glu Cys Cys Asn Ser Lys
    130                 135                 140